# EUROPEAN PATENT APPLICATION

(11) **EP 4 850 717 A1**
(43) Date of publication of application: **09.12.2026**
(21) Application number: 25747960.0
(22) Date of filing: 26.01.2025
(51) Int. Cl.: A61M 5/14

(54) **FLUID CONVEYING DEVICE**

(30) Priority: 01.02.2024 CN 202410146195
(71) Applicant: SHENZHEN SISENSING CO., LTD., Shenzhen Guangdong 518110 (CN)
(72) Inventor: XIA, Bin, Shenzhen, Guangdong 518110 (CN)
(74) Representative: Vossius & Partner Patentanwälte Rechtsanwälte mbB
(86) International application number: PCT/CN2025/075251
(87) International publication number: WO 2025/162396

(57) **Abstract**

A fluid delivery device, including a fluid channel, an actuation component, and a flow limiting component. The fluid channel includes a first channel, a reservoir, and a second channel, which are in fluid communication with each other. The actuation component is configured to provide actuating force to make fluid flow into or flow out of the reservoir. The flow limiting component is configured to open or close the first channel and/or the second channel. According to this disclosure, a fluid delivery device capable of quantitatively delivering fluid is provided.

## Description

This disclosure relates to the field of biomedical engineering, specifically to a fluid delivery device.

### BACKGROUND

Chronic diseases are characterized by prolonged course, slow progression, and difficulty in cure. They are typically related to multiple factors, including genetics, environment, lifestyle, and so on, and often require long-term management and treatment. There are many types of chronic diseases, such as diabetes, cardiovascular disease, chronic kidney disease and the like. Taking diabetes as an example, patients generally treat their diseases by using an insulin pump. The insulin pump simulates the physiological pattern of insulin secretion in the human pancreas, and quantitatively delivers insulin to the patient's body at regular intervals to stabilize blood sugar.

Because patients usually need to quantitatively receive insulin injections at regular intervals, existing insulin pumps are generally portable, which are convenient for patients to carry around or apply to their bodies. Portable insulin pumps typically have a cartridge, a piston, and a tube for drug delivery. When medication is needed, the medication in the cartridge can be pushed by the piston to deliver it to the body through the tube, and the dosage is controlled by adjusting the pushing distance of the piston.

However, existing portable insulin pumps often experience backflow or leakage during use, which makes it difficult to maintain a stable insulin dose infused into the patient's body. In addition, most existing portable insulin pumps rely on complex mechanical structures to pump out a certain dose of insulin, which results in low reliability and portability, and the energy consumption of insulin pumps is increased by driving complex mechanical structures.

### SUMMARY

This disclosure is made in view of the aforementioned situation of the prior art, and its object is to provide a fluid delivery device capable of quantitatively delivering fluid.

To this end, this disclosure provides a fluid delivery device, including a fluid channel, an actuation component, and a flow limiting component, the fluid channel includes a first channel, a reservoir, and a second channel, which are in fluid communication with each other, the actuation component is configured to provide actuating force to make the fluid flow into or flow out of the reservoir, and the flow limiting component is configured to open or close the first channel and/or the second channel.

In this disclosure, a path for fluid flow can be provided by the first channel, the reservoir, and the second channel, which are in fluid communication with each other. Furthermore, the actuation component provides actuating force, which enables the fluid to flow within the fluid channel and facilitates the flow of a preset volume of the fluid into or out of the reservoir. Additionally, the flow limiting component opens and closes the first channel and/or the second channel, which facilitates the fluid flow along a desired path.

Additionally, in the fluid delivery device of this disclosure, optionally, the first channel, the reservoir, and the second channel are connected sequentially. Thus, the fluid can flow into the reservoir through the second channel and flow out of the reservoir through the first channel; or the fluid can flow into the reservoir through the first channel and flow out of the reservoir through the second channel.

Additionally, in the fluid delivery device of this disclosure, optionally, the first channel, the reservoir, and the second channel are integrally formed. In this case, since the first channel, the reservoir, and the second channel are integrally formed, the gap between the components can be reduced compared to a separate fluid channel, thereby reducing the possibility of fluid leakage in the fluid channel.

Additionally, in the fluid delivery device of this disclosure, optionally, the actuation component includes a power source configured to provide the actuating force. Thus, the actuation component can provide the actuating force.

Additionally, in the fluid delivery device of this disclosure, optionally, the actuating force acts on the reservoir, and the reservoir actuates the fluid in the reservoir after being subjected to the force.

Additionally, in the fluid delivery device of this disclosure, optionally, the actuating force acts on the fluid.

Additionally, in the fluid delivery device of this disclosure, optionally, the actuating force is configured to increase the pressure in the reservoir to make the fluid flow out of the reservoir.

Additionally, in the fluid delivery device of this disclosure, optionally, the reservoir is deformed to reduce its volume. In this case, since the reduced volume of the reservoir is the volume of the fluid flowing out of the reservoir, reducing the volume of the reservoir helps to precisely control the volume of the fluid flowing out of the reservoir.

Additionally, in the fluid delivery device of this disclosure, optionally, the actuating force is configured to reduce the pressure in the reservoir to make the fluid flow into the reservoir.

Additionally, in the fluid delivery device of this disclosure, optionally, the reservoir is deformed to increase its volume. In this case, since the increased volume of the reservoir is the volume of the fluid flowing into the reservoir, increasing the volume of the reservoir helps to precisely control the volume of the fluid flowing into the reservoir.

Additionally, in the fluid delivery device of this disclosure, optionally, the actuating force acts on the reservoir to increase or decrease its volume.

Additionally, in the fluid delivery device of this disclosure, optionally, the reservoir is made of an elastic material. Thus, the reservoir can increase or decrease its volume by utilizing the properties of the elastic material.

Additionally, in the fluid delivery device of this disclosure, optionally, the flow limiting component restricts the flow of the fluid in the first channel and/or the second channel.

Additionally, in the fluid delivery device of this disclosure, optionally, the flow limiting component includes a valve disposed on the flow path of the fluid in the first channel and/or the second channel.

Additionally, in the fluid delivery device of this disclosure, optionally, the flow limiting component acts on the first channel and/or the second channel to close or open the flow path of the fluid.

Additionally, in the fluid delivery device of this disclosure, optionally, the actuation component provides the actuating force in response to the flow limiting component opening the first channel and/or the second channel. In this case, by first connecting the reservoir to the first channel and/or the second channel, and then providing the actuating force through the actuation component, the utilization rate of the actuating force can be improved.

Additionally, in the fluid delivery device of this disclosure, optionally, the flow limiting component alternately opens the first channel and the second channel. In this case, by alternately opening the first channel and the second channel, the fluid can flow into or out of the reservoir through one of the first channel and the second channel, thereby enabling directional fluid flow.

Additionally, in the fluid delivery device of this disclosure, optionally, a holding component is comprised, and the holding component is configured to hold the position of the actuation component and provide the actuating force.

According to this disclosure, a fluid delivery device capable of quantitatively delivering fluid is provided.

### BRIEF DESCRIPTION OF THE DRAWINGS

This disclosure will now be further explained in detail only by referring to examples in the accompanying drawings.
Figure 1 is a schematic diagram illustrating an application scenario of the fluid delivery device according to an example of this disclosure.
Figure 2 is a structural schematic diagram illustrating the fluid delivery device according to an example of this disclosure.
Figure 3A is a schematic diagram illustrating an actuator acting on the reservoir and causing deformation of the reservoir according to an example of this disclosure.
Figure 3B is a schematic diagram illustrating the reservoir returning to its original shape according to an example of this disclosure.
Figure 3C is a schematic diagram illustrating another embodiment of the actuator according to an example of this disclosure.
Figure 3D is a schematic diagram illustrating another embodiment of the actuator according to an example of this disclosure.
Figure 4A is a schematic diagram illustrating a first lever located at a third preset position according to an example of this disclosure.
Figure 4B is a schematic diagram illustrating the first lever located at a fourth preset position according to an example of this disclosure.
Figure 5A is a schematic diagram illustrating a second lever located at the third preset position according to an example of this disclosure.
Figure 5B is a schematic diagram illustrating the second lever located at the fourth preset position according to an example of this disclosure.
Figure 6A is a schematic diagram illustrating the reservoir supplying the fluid when the valve is a passive valve according to an example of this disclosure.
Figure 6B is a schematic diagram illustrating the reservoir receiving the fluid when the valve is the passive valve according to an example of this disclosure.
Figure 7 is a schematic diagram illustrating a first position-limiting portion according to an example of this disclosure.
Figure 8A is a schematic diagram illustrating a first holder actuating a first end according to an example of this disclosure.
Figure 8B is a schematic diagram illustrating a second holder actuating a second end according to an example of this disclosure.
Figure 9A is a schematic diagram illustrating a puncture mechanism entering a target in a first embodiment of an application component according to an example of this disclosure.
Figure 9B is a schematic diagram illustrating the puncture mechanism withdrawing from the target in the first embodiment of the application component according to an example of this disclosure.
Figure 10A is a schematic diagram illustrating the puncture mechanism before entering the target in a second embodiment of the application component according to an example of this disclosure.
Figure 10B is a schematic diagram illustrating the puncture mechanism entering the target in the second embodiment of the application component according to an example of this disclosure.
Figure 10C is a schematic diagram illustrating the puncture mechanism withdrawing from the target in the second embodiment of the application component according to an example of this disclosure.
Figure 11A is a schematic diagram illustrating the puncture mechanism before entering the target in a third embodiment of the application component according to an example of this disclosure.
Figure 11B is a schematic diagram illustrating the puncture mechanism entering the target in the third embodiment of the application component according to an example of this disclosure.
Figure 11C is a schematic diagram illustrating the puncture mechanism withdrawing from the target in the third embodiment of the application component according to an example of this disclosure.
Figure 11D is a schematic diagram illustrating a drive source in the third embodiment of the application component according to an example of this disclosure.
Figure 12A is a schematic diagram illustrating the puncture mechanism entering the target in a fourth embodiment of the application component according to an example of this disclosure.
Figure 12B is a schematic diagram illustrating the puncture mechanism withdrawing from the target in the fourth embodiment of the application component according to an example of this disclosure.
Figure 13A is a schematic diagram illustrating the puncture mechanism entering the target in a fifth embodiment of the application component according to an example of this disclosure.
Figure 13B is a schematic diagram illustrating the puncture mechanism withdrawing from the target in the fifth embodiment of the application component according to an example of this disclosure.
Figure 14A is a schematic diagram illustrating the puncture mechanism entering the target in a sixth embodiment of the application component according to an example of this disclosure.
Figure 14B is a schematic diagram illustrating the puncture mechanism withdrawing from the target in the sixth embodiment of the application component according to an example of this disclosure.

### Description of Reference Numerals

1...fluid delivery device, 11...adhesive sheet, 12...fluid channel, 121...first channel, 122...reservoir, 123...second channel, 13...actuation component, 131...actuator, 1311...actuating portion, 1312...first end, 1313...second end, 132...first lever, 1321...first mating portion, 133...second lever, 1331...second mating portion, 134...power source, 1341...first power component, 1342...second power component, 1343...pulley, 14...flow limiting component, 141...first valve, 1411...first opening and closing member, 1412...first engaging portion, 1413...first position-limiting portion, 142...second valve, 1421...second opening and closing member, 1422...second engaging portion, 15...substrate, 151...first fixing portion, 152...second fixing portion, 153...third fixing portion, 16...reset component, 161...first reset member, 162...second reset member, 17...holding component, 171...first holding member, 172...second holding member, 18...application component, 181...drive mechanism, 1811...drive source, 1812...transmission member, 182...puncture mechanism, 183...guide mechanism, 2...target, A...central fulcrum, P1...first fulcrum, P2...second fulcrum, CA...central axis.

### DETAILED DESCRIPTION OF EMBODIMENTS

Hereinafter, preferred embodiments of the present disclosure will be described in detail with reference to the accompanying drawings. In the following description, the same reference numerals are used for the same parts, and repeated descriptions are omitted. Furthermore, the drawings are merely schematic diagrams, and the proportions of the dimensions of the parts or the shapes of the parts may differ from actual ones.

It should be noted that the terms "including" and "having" and any variations thereof, in this disclosure, refer to a process, method, system, product, or device that includes or has a series of steps or units, not necessarily limited to those steps or units explicitly listed, but may include or have other steps or units not explicitly listed or inherent to the process, method, product, or device.

It should be noted that in this document, relative position and direction terms such as "above," "facing upward," "below," "facing downward," "up and down direction," "left side," "facing left," "left," "facing left," "right side," "facing right," "right," "facing right," "left and right direction," "front," "facing forward," "rear," "facing backward," and "front and back direction" are used with reference to a general operating posture and should not be considered restrictive.

In this disclosure, unless otherwise expressly specified and limited, connection can be understood as a mechanical connection. For example, "connection" can refer to a fixed connection, a detachable connection, or an integral connection; it can be a direct connection or an indirect connection through intermediate components.

In some examples, leakage can refer to an undesirable fluid leak in the fluid channel, which may cause the actual volume of fluid delivered by the fluid delivery device to be less than the preset volume. Taking insulin as an example, the amount of insulin delivered by the fluid delivery device is insufficient, which may affect the health status of the host (also known as the patient or user). Therefore, reducing the possibility of leakage can help maintain the host's health. In addition to reducing the possibility of leakage, other means that help the fluid delivery device deliver a predetermined volume of fluid can also help maintain the host's health.

This disclosure relates to a fluid delivery device for delivering fluid to a target. The fluid delivery device according to this disclosure is characterized by its small size, low cost, simple structure, and portability. Meanwhile, the fluid delivery device according to this disclosure enables more accurate delivery of the volume of fluid to the target. The fluid delivery device according to this disclosure may also be referred to as a drug pump, drug delivery device, fluid delivery pump, or fluid infusion device, etc.

In some examples, the fluid delivery device according to this disclosure may also be referred to as a delivery device, fluid transport device, fluid dispensing device, fluid injection device, fluid pumping device, pump, drug pump, insulin pump, drug delivery device, or insulin delivery device, etc.

In some examples, the fluid according to this disclosure may refer to a drug delivered through the fluid delivery device according to this disclosure. For example, the fluid may be any one or a combination of hormones, antitoxins, analgesics, peptides, proteins, insulin, enzymes, oligonucleotides, anti-allergic agents, antihistamines, anti-inflammatory agents, corticosteroids, erythropoietin, or vaccines. In some examples, the fluid may be dopamine, dobutamine, adrenaline, sodium nitroprusside, somafostatin, propofol, insulin, or glucagon-like peptide-1, etc.

Hereinafter, with reference to the accompanying drawings, the fluid delivery device and fluid delivery system including the fluid delivery device according to this disclosure will be described in detail.

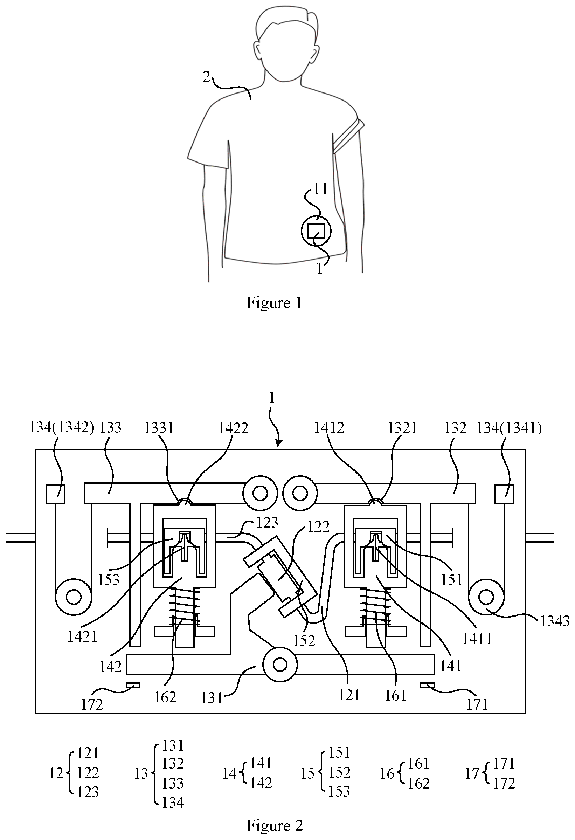

Figure 1 is a schematic diagram illustrating an application scenario of the fluid delivery device 1 according to an example of this disclosure.

In some examples, the fluid delivery device 1 can be configured to deliver fluid. In some examples, the fluid delivery device 1 can deliver fluid from the inside of the fluid delivery device 1 to the outside. In some examples, the fluid delivery device 1 can deliver fluid into the body of a target 2. In some examples, the fluid can be pre-stored within the fluid delivery device 1. In this case, since the fluid delivery device 1 can directly deliver fluid into the body of the target 2, the convenience and timeliness of the target 2 obtaining fluid can be improved. In some examples, the target 2 can also be referred to as a host, patient, or user.

In other examples, the fluid delivery device 1 can also deliver fluid from the outside of the fluid delivery device 1 to the inside. In some examples, the fluid delivery device 1 can also deliver fluid from inside the body of the target 2 to the outside of the target 2. For example, the fluid delivery device 1 can also collect fluid from inside the body of the target 2.

In some examples, the fluid delivery device 1 may be disposed on the target 2 (see Figure 1). In some examples, the fluid delivery device 1 may be disposed on the body surface of the target 2 or inside the body of the target 2. In some examples, a part of the fluid delivery device 1 may be disposed on the body surface of the target 2, and another part may be disposed inside the body of the target 2. In other examples, the fluid delivery device 1 may be disposed entirely inside the body of the target 2.

In some examples, the fluid delivery device 1 can be applied to the target 2. In some examples, the fluid delivery device 1 can be applied to the body surface of the target 2. In some examples, the fluid delivery device 1 can be applied to the target 2 by its own structure (e.g., an application component 18 described later).

In some examples, the fluid delivery device 1 can be fixed to the target 2. In some examples, the fluid delivery device 1 can be fixed to the body surface of the target 2. For example, the fluid delivery device 1 can be fixed to the abdomen or arm of the target 2. However, this disclosure is not limited to this, and the fixing position of the fluid delivery device 1 can be adjusted according to needs. In some examples, the fluid delivery device 1 can be adhered to the target 2. In some examples, the fluid delivery device 1 can also be tied to the target 2 by a strap.

In some examples, referring to Figure 1, the fluid delivery device 1 can be adhered to the body surface of the target 2. In some examples, the fluid delivery device 1 can include an adhesive sheet 11 with adhesive properties. In some examples, the fluid delivery device 1 can be adhered to the body surface of the target 2 by the adhesive sheet 11. Thus, it is convenient to fix the fluid delivery device 1 to the target 2.

In some examples, at least a part of the fluid delivery device 1 can be placed inside the target 2. For example, it can be placed subcutaneously in the target 2. Thus, it facilitates the fluid delivery device 1 to deliver fluid into the body of the target 2. In some examples, when the fluid delivery device 1 is placed inside the target 2, the fluid delivery device 1 can deliver fluid into the target 2.

Figure 2 is a structural schematic diagram illustrating the fluid delivery device 1 according to an example of this disclosure.

In some examples, the fluid delivery device 1 may include a fluid channel 12 and an actuation component 13 (see Figure 2). In some examples, the fluid channel 12 can store fluid. In some examples, at least a part of the fluid channel 12 can be placed inside the target 2, and the actuation component 13 can provide actuating force to deliver fluid into the target 2.

In some examples, the fluid delivery device 1 may also include a flow limiting component 14 (see Figure 2). In some examples, the flow limiting component 14 can restrict the flow of fluid in the fluid channel 12. In some examples, the flow limiting component 14 can be configured to open or close the fluid channel 12. Thus, it is possible to restrict the direction of the fluid flow.

In some examples, referring to Figure 2, the fluid delivery device 1 may include a substrate 15. In some examples, the substrate 15 can be configured to support the fluid channel 12, the actuation component 13, and the flow limiting component 14. In some examples, the fluid channel 12, the actuation component 13, and the flow limiting component 14 can be disposed on the substrate 15.

In some examples, the substrate 15 can be plate-shaped. In some examples, the substrate 15 can have two surfaces. In some examples, one surface of the substrate 15 can be provided with the fluid channel 12, the flow limiting component 14 and the actuation component 13, and the other surface of the substrate 15 can be adhered to the body surface of the target 2. In some examples, the substrate 15 can also have other shapes and structures that facilitate the installation of components.

In some examples, referring to Figure 2, at least a part of the fluid channel 12 can extend through the substrate 15. Thus, it can facilitate the entry of the fluid channel 12 into the body of the target 2.

In some other examples, the fluid channel 12 may also be all located on the same surface of the substrate 15.

In some examples, referring to Figure 2, the fluid delivery device 1 may include the fluid channel 12. In some examples, the fluid channel 12 may be configured to store fluid.

In some examples, fluid may be pre-stored in the fluid channel 12. Thus, it allows the fluid delivery device 1 to deliver fluid more timely.

In some examples, fluid may be replenished into the fluid delivery device 1. In some examples, fluid may be replenished into the fluid channel 12.

In some examples, the fluid channel 12 may also be configured to provide a flow path. In some examples, fluid may flow into and/or out of the target 2 via the fluid channel 12. In some examples, the fluid channel 12 may guide fluid flow into and/or out of the target 2.

In some examples, fluid can be replenished into the fluid delivery device 1 through the fluid channel 12.

In some examples, the fluid channel 12 may be at least partially placed at target 2. In some examples, the fluid channel 12 may be at least partially placed within the body of the target 2. Thus, it facilitates the delivery of fluid into the body of the target 2.

In some examples, referring to Figure 2, the fluid channel 12 may include a first channel 121. In some examples, fluid may flow through the first channel 121. In some examples, fluid may flow into or out of the fluid delivery device 1 via the first channel 121.

In some examples, fluid may flow out through one end of the first channel 121 and flow in through the other end of the first channel 121.

In some examples, at least a part of the first channel 121 may be placed within the body of the target 2. In some examples, one end of the first channel 121 may be implanted into the target 2.

In some examples, the fluid outflow end of the first channel 121 may be placed within the body of the target 2. In some examples, fluid may be delivered to the target 2 via the first channel 121.

In some examples, the first channel 121 can be a tubular structure (see Figure 2). In some examples, the inner diameter of the first channel 121 can be from 0.1 mm to 1.5 mm. For example, the inner diameter of the first channel 121 can be 0.1 mm, 0.2 mm, 0.3 mm, 0.5 mm, 0.7 mm, 1 mm, or 1.5 mm, etc. Preferably, the inner diameter of the first channel 121 can be 0.3 mm.

In some examples, the outer diameter of the first channel 121 can be from 0.2 mm to 2 mm. For example, the outer diameter of the first channel 121 can be 0.2 mm, 0.3 mm, 0.4 mm, 0.5 mm, 0.7 mm, 1 mm, 1.5 mm, or 2 mm, etc. Preferably, the outer diameter of the first channel 121 can be 1 mm.

In some examples, the first channel 121 may be cylindrical. In some examples, the cross-sectional shape of the first channel 121 may be circular. Thus, it helps to disperse the pressure on the first channel 121, thereby reducing the possibility of deformation of the first channel 121.

In other examples, the first channel 121 may also be prismatic. In some examples, the cross-sectional shape of the first channel 121 may also be square, star-shaped, or other irregular shapes.

In some examples, the first channel 121 may be made of an elastic material. For example, the first channel 121 may be made of at least one of silicone, rubber, and elastomeric polymers. In this case, the first channel 121 can be easily bent and shaped, thereby improving the flexibility of the first channel 121 during installation and layout.

In some examples, the first channel 121 may be made of a biocompatible material. In some examples, the biocompatible material used to form the first channel 121 may include, but is not limited to, medical-grade polyvinyl chloride (PVC), polyethylene (PE), polyether ether ketone (PEEK), polycarbonate (PC), polyetherimide (PEI), polysulfone (PSF), polypropylene (PP), polyurethane (PU), and thermoplastic polyurethane elastomer (TPU).

Additionally, in some examples, the first channel 121 may be made of a hard metal. For example, the first channel 121 may be made of at least one of stainless steel (e.g., 304 stainless steel, 316 stainless steel, etc.), titanium alloy, and aluminum. In some examples, the first channel 121 may be made of a hard non-metallic material. For example, the first channel 121 may be made of at least one of glass, plastic, ceramic composites, and carbon fiber composites. In this case, the first channel 121 is less prone to breakage, thereby ensuring more stable fluid flow.

In some examples, the inner wall of the first channel 121 may be coated with a lubricating layer. In some examples, the lubricating layer may be made of polytetrafluoroethylene (PTFE). Thus, it facilitates fluid flow through the first channel 121, thereby helping to prevent blockage of the first channel 121.

In some examples, referring to Figure 2, the fluid channel 12 may include a reservoir 122. In some examples, fluid may flow through the reservoir 122. In some examples, fluid may flow into or out of the fluid delivery device 1 via the reservoir 122.

In some examples, fluid may flow out through one end of the reservoir 122 and flow in through the other end of the reservoir 122. In some examples, the fluid outflow end of the reservoir 122 may be connected to the fluid inflow end of the first channel 121.

In some examples, the reservoir 122 may be configured to receive fluid. In some examples, receiving fluid in the reservoir 122 may refer to fluid flowing into the reservoir 122. In some examples, the manner that the reservoir 122 receives fluid may include at least one of inflow, extrusion, suction, and pumping.

In some examples, for ease of understanding, receiving fluid may also be referred to as replenishing fluid; for example, receiving fluid in the reservoir 122 may be referred to as replenishing fluid to the reservoir 122; providing fluid may also be referred to as transporting fluid; for example, providing fluid by the reservoir 122 may be referred to as delivering fluid from the reservoir 122 to the target 2.

In some examples, the reservoir 122 may also be configured to provide fluid. In some examples, providing fluid by the reservoir 122 may refer to fluid flowing out of the reservoir 122, or may be referred to as fluid being discharged from the reservoir 122. In some examples, fluid flowing out of the reservoir 122 may flow into the first channel 121. In some examples, the fluid in the reservoir 122 can be delivered to the target 2 via the first channel 121.

In some examples, the reservoir 122 can also be configured to store fluid. In some examples, fluid flowing into the reservoir 122 can be stored in the reservoir 122. In some examples, fluid delivered to the target 2 can be pre-stored in the reservoir 122.

In some examples, at least a part of the reservoir 122 can be located outside the target 2.

In some examples, the reservoir 122 can be a tubular structure. In some examples, the diameter of the two end portions of the reservoir 122 can be equal to or smaller than the diameter of the middle portion. For example, the reservoir 122 can be a spindle-shaped structure. In other examples, the diameter of the two end portions of the reservoir 122 can be larger than the diameter of the middle portion.

In some examples, the reservoir 122 can be made of an elastic material. Thus, it is possible to easily increase or decrease the volume of the reservoir 122. A detailed description of the material of the reservoir 122 can be found in the description of the first channel 121, and will not be repeated here.

In some examples, the reservoir 122 may have at least one dispensing port. For example, the reservoir 122 may have one, two, or three dispensing ports. In some examples, the dispensing port can be used to replenish fluid to the reservoir 122.

In some examples, referring to Figure 2, the fluid channel 12 may include the second channel 123. In some examples, fluid may flow through the second channel 123. In some examples, fluid may flow into or out of the fluid delivery device 1 via the second channel 123.

In some examples, fluid may flow out through one end of the second channel 123 and flow in through the other end of the second channel 123. In some examples, the fluid outflow end of the second channel 123 may be connected to the fluid inflow end of the reservoir 122.

In some examples, the second channel 123 may be configured to replenish fluid. In some examples, fluid may be replenished into the fluid delivery device 1 via the second channel 123. In some examples, fluid may flow into the reservoir 122 via the second channel 123. In some examples, replenishing fluid via the second channel 123 may mean that fluid flows into the second channel 123 and is stored in the second channel 123, or that fluid flows into via the second channel 123 and is stored in the reservoir 122.

In some other examples, fluid can be replenished into the fluid delivery device 1 via the first channel 121, or it can be delivered to the target 2 via the second channel 123.

In some examples, the second channel 123 can be located outside the target 2. Thus, it can facilitate the replenishment of fluid into the fluid delivery device 1.

The description of the structure and material of the second channel 123 can refer to the relevant description of the first channel 121, and will not be repeated here.

In some examples, the fluid channel 12 can include a drug reservoir. In some examples, the drug reservoir can be configured to pre-store fluid. In some examples, the fluid that needs to be delivered to the target 2 can be pre-stored in the drug reservoir. Thus, it facilitates timely access to fluid for the target 2.

In some examples, the volume of the drug reservoir can be larger than the volume of the reservoir 122. Thus, it facilitates increasing the storage capacity of the fluid delivery device 1. In other examples, the volume of the drug reservoir can be equal to or smaller than the volume of the reservoir 122.

In some examples, the drug reservoir can discharge fluid. In some examples, the fluid discharged from the drug reservoir can enter the second channel 123. In some examples, the fluid discharged from the drug reservoir can be delivered to the reservoir 122. In some examples, the drug reservoir can discharge fluid by being applied an action. For example, the drug reservoir can discharge fluid by being squeezed. In some examples, the volume of the fluid discharged from the drug reservoir can be a preset volume.

In some examples, the fluid delivery device 1 can include a piston. In some examples, the piston can be connected to the drug reservoir. In some examples, pressure can be applied to the fluid in the drug reservoir by moving the piston to discharge the fluid from the drug reservoir.

In some examples, when the piston applies a preset pressure to the fluid in the drug reservoir, the drug reservoir can discharge a preset volume of fluid. In some examples, the preset pressure and preset volume can be adjusted as needed. In this case, since the volume of the fluid discharged from the drug reservoir is related to the pressure, when the pressure is adjusted to the preset pressure, the drug reservoir can discharge a preset volume of fluid.

In some examples, the drug reservoir may be equipped with a pressure sensor. Thus, it allows the pressure of the fluid in the drug reservoir to be obtained by the pressure sensor when the piston applies the pressure to the drug reservoir.

In other examples, the sensor in the drug reservoir may also be a capacitive sensor. In some examples, the volume of the remaining fluid in the drug reservoir can be detected by measuring the capacitance between the piston and the end of the drug reservoir from which the fluid is discharged.

In other examples, the fluid delivery device 1 may include a spring. In some examples, the spring may abut against the drug reservoir. In some examples, the spring may act on the drug reservoir. In some examples, the drug reservoir may discharge fluid under the action of the spring.

In other examples, the drug reservoir may also be a vacuum soft bag. In this case, since the drug reservoir is more deformable, its shape can be arbitrarily changed based on the structure of the fluid delivery device 1, thereby helping to improve the space utilization of the fluid delivery device 1 and facilitate the miniaturization of the fluid delivery device 1.

In other examples, the drug reservoir may also have a structure that is generally in the form of a flexible hose. Thus, it facilitates the drainage of fluid from the drug reservoir.

In some examples, the drug reservoir may have at least one dispensing port. For example, the drug reservoir may have one, two, or three dispensing ports. In some examples, the dispensing port can be used to replenish fluid to the drug reservoir.

In some examples, when the drug reservoir stores fluid, the pressure of the fluid within the drug reservoir can be maintained within a preset range. In this case, the occurrence of negative pressure or air bubbles due to the emptying of fluid from the drug reservoir can be reduced, thereby improving the stability of the drug reservoir.

The description of the material of the drug reservoir can refer to the relevant description of the first channel 121, and will not be repeated here.

In some other examples, when the fluid channel 12 has the first channel 121, the first channel 121 can receive, store, and supply fluid to the target 2; when the fluid channel 12 has the first channel 121 and the reservoir 122, the reservoir 122 can receive, store, and supply fluid to the first channel 121, and the first channel 121 can receive and supply fluid to the target 2; when the fluid channel 12 has the first channel 121, the reservoir 122, and the second channel 123, the second channel 123 can receive and supply fluid to the reservoir 122, the reservoir 122 can receive, store, and supply fluid to the first channel 121, and the first channel 121 can receive and supply fluid to the target 2.

In some examples, referring to Figure 2, the first channel 121 can be connected to the reservoir 122. In some examples, the first channel 121 can be fixedly connected to the reservoir 122. In some examples, the first channel 121 can be integrally formed with the reservoir 122.

In some examples, the connection method between the first channel 121 and the reservoir 122 can be at least one of fusion welding and bonding.

In other examples, the first channel 121 can also be detachably connected to the reservoir 122.

In some examples, the first channel 121 can be connected to any location on the reservoir 122. In some examples, the first channel 121 and the reservoir 122 can be in fluid communication.

As described above, the first channel 121 can be at least partially placed inside the body of the target 2. In some examples, the end of the first channel 121 which is not placed inside the body of the target 2 can be connected to the reservoir 122.

In some examples, referring to Figure 2, the second channel 123 can be connected to the reservoir 122. In some examples, the second channel 123 can be fixedly connected to the reservoir 122. In some examples, the second channel 123 can be integrally formed with the reservoir 122.

In some examples, the connection method between the second channel 123 and the reservoir 122 can be at least one of fusion welding and bonding.

In other examples, the second channel 123 can also be detachably connected to the reservoir 122.

In some examples, the second channel 123 can be connected to any position on the reservoir 122. In some examples, the second channel 123 and the reservoir 122 can be in fluid communication.

In some examples, the first channel 121, the reservoir 122 and the second channel 123 can be integrally formed. In this case, since the first channel 121, the reservoir 122 and the second channel 123 are integrally formed, the gaps between the components can be reduced compared to a separate fluid channel 12, thereby decreasing the possibility of leakage from the fluid channel 12.

In some examples, the first channel 121, the reservoir 122, and the second channel 123 can be connected sequentially. Thus, it allows fluid to flow into the reservoir 122 through the second channel 123 and flow out of the reservoir 122 through the first channel 121; or fluid to flow into the reservoir 122 through the first channel 121 and flow out of the reservoir 122 through the second channel 123.

In some examples, the first channel 121, the reservoir 122, and the second channel 123 can be connected end-to-end sequentially. In some examples, the first channel 121 and the second channel 123 can be connected to any position on the reservoir 122. In some examples, the connection method of the first channel 121, the reservoir 122, and the second channel 123 can be at least one of fusion welding and bonding.

In some examples, the first channel 121 may be connected to the second channel 123. In some examples, the first channel 121 may be connected to the reservoir 122. In some examples, the second channel 123 may be connected to the reservoir 122.

In some examples, the first channel 121, the reservoir 122, and the second channel 123 may be interconnected. In some examples, the first channel 121, the reservoir 122, and the second channel 123 may be in fluid communication. Thus, it provides a path for fluid flow. In some examples, fluid communication may mean that the first channel 121, the reservoir 122, and the second channel 123 are connected and fluid can flow between the first channel 121, the reservoir 122, and the second channel 123.

In some examples, the drug reservoir may be connected to the reservoir 122. In some examples, the drug reservoir may be in fluid communication with the reservoir 122. In some examples, the drug reservoir can communicate with the reservoir 122 via the second channel 123. In some examples, the drug reservoir, the second channel 123, and the reservoir 122 can be connected sequentially. In some examples, the drug reservoir, the second channel 123, and the reservoir 122 can be integrally formed.

In some examples, referring to Figure 2, the substrate 15 may include a first fixing portion 151 which can be configured to fix the first channel 121.

In some examples, the first fixing portion 151 can be formed on the substrate 15. In some examples, the first fixing portion 151 can be a protrusion or groove formed on the substrate 15. In some examples, the first fixing portion 151 can have a fixing groove. In some examples, the first channel 121 can be fixed to the fixing groove. In some examples, the number of fixing grooves can be multiple. For example, there can be 2, 4, or 6. Preferably, the first fixing portion 151 can have 2 fixing grooves. Thus, it allows for more stable fixing of the first channel 121.

In some examples, the size of the fixing groove on the first fixing portion 151 may be equal to or smaller than the size of the first channel 121. Thus, it allows for more stable fixing of the first channel 121. In other examples, the size of the fixing groove may be larger than the size of the first channel 121.

In other examples, the substrate 15 may not have the first fixing portion 151. In some examples, the first channel 121 may be fixed to the substrate 15 by one or more of suction, bonding, and fusion welding.

In some examples, referring to Figure 2, the substrate 15 may further include the second fixing portion 152 which may be configured to fix the reservoir 122. In some examples, referring to Figure 2, the substrate 15 may include the third fixing portion 153 which may be configured to fix the second channel 123. The descriptions regarding the second fixing portion 152 fixing the reservoir 122 and the third fixing portion 153 fixing the second channel 123 can refer to the relevant description of the first fixing part 151 fixing the first channel 121, and will not be repeated here.

For ease of understanding, the following description uses the example of fluid flowing out of the fluid delivery device 1 via the first channel 121 (i.e., the first channel 121 is the liquid outlet channel) and fluid flowing into the fluid delivery device 1 via the second channel 123 (i.e., the second channel 123 is the liquid inlet channel). However, it should be noted that the above definitions are intended to more clearly illustrate the present disclosure and should not be construed as limiting the present disclosure. The descriptions of the present disclosure also apply to the case where the first channel 121 is the liquid inlet channel and the second channel 123 is the liquid outlet channel.

In some examples, the fluid in the fluid channel 12 can be actuated to be delivered into the body of the target 2. In some examples, the fluid can be actuated by an actuating force. In some examples, the fluid can be actuated by one or more of squeezing, pushing, suction, and traction.

In some examples, referring to Figure 2, the fluid delivery device 1 may include an actuation component 13. In some examples, the actuation component 13 may be configured to actuate fluid. Thus, it enables fluid to flow in the fluid channel 12 and facilitates the flow of a predetermined volume of fluid into or out of the reservoir 122. In some examples, the actuation component 13 may be configured to provide the actuating force to make fluid flow into or flow out of the reservoir 122. In some examples, the actuation component 13 may actuate fluid in the fluid channel 12, and the actuation component 13 may also actuate fluid within the target 2.

In some examples, the actuation component 13 may act on the fluid. In some examples, the actuating force may act on the fluid. For example, the actuation component 13 may be a piston in the fluid channel 12. Thus, the actuation component 13 can directly actuate the fluid.

In some examples, the actuation component 13 may act on the fluid channel 12. In some examples, the actuating force may act on the fluid channel 12. In some examples, the fluid channel 12 can actuate the fluid within the fluid channel 12 under the action of the actuating force. Thus, the actuation component 13 can actuate the fluid through the fluid channel 12.

In some examples, referring to Figure 2, at least a part of the actuation component 13 can be disposed in the fluid channel 12. Thus, it facilitates the actuation component 13 acting on the fluid channel 12.

In some examples, the actuating force can be configured to increase the pressure in the reservoir 122. Thus, it facilitates the outflow of fluid from the reservoir 122.

In some examples, the actuating force can also be configured to decrease the pressure in the reservoir 122. Thus, it facilitates the inflow of fluid into the reservoir 122.

In some examples, the reservoir 122 can deform. In some examples, the reservoir 122 can deform under the action of the actuating force.

In some examples, the actuating force can be applied directly to the reservoir 122 in order to cause the reservoir 122 to deform. In some examples, the reservoir 122 can be deformed to reduce its volume. In some examples, the deformation of the reservoir 122 can be equal to the reduced volume. In some examples, when the reservoir 122 deforms to reduce its volume, fluid can flow out of the reservoir 122. For example, the reservoir 122 can be recessed towards its interior space to compress the fluid, allowing the fluid to flow out of the reservoir 122 under this compression. Thus, it helps to precisely control the volume of fluid flowing out of the reservoir 122.

In some examples, the reservoir 122 can be deformed to increase its volume. In some examples, the deformation of the reservoir 122 can be equal to the increased volume. In some examples, when the reservoir 122 deforms to increase its volume, fluid can flow into the reservoir 122. For example, the reservoir 122 can expand toward the external space to draw in fluid, and the liquid can flow into the reservoir 122 under the suction. Thus, it helps to precisely control the volume of fluid flowing into the reservoir 122.

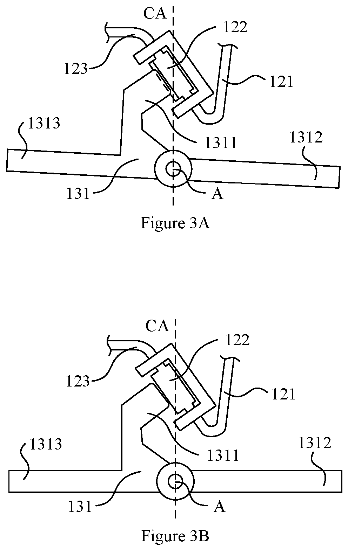

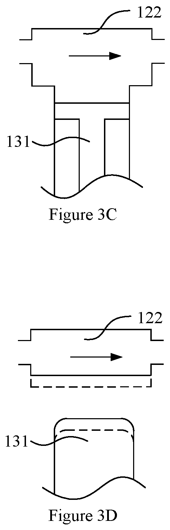

Figure 3A is a schematic diagram illustrating an actuator 131 acting on the reservoir 122 and deforming it according to an example of this disclosure. Figure 3B is a schematic diagram illustrating the reservoir 122 returning to its original shape according to an example of this disclosure. Figure 3C is a schematic diagram illustrating another embodiment of the actuator 131 according to an example of this disclosure. Figure 3D is a schematic diagram illustrating another embodiment of the actuator 131 according to an example of this disclosure.

In some examples, referring to Figure 3A or Figure 3B, the actuation component 13 may include the actuator 131. In some examples, the actuator 131 may be configured to actuate fluid. In some examples, the actuator 131 may provide the actuating force.

In some examples, the actuator 131 can act on the fluid.

In some examples, the actuator 131 can act on the fluid channel 12.

In some examples, at least a part of the actuator 131 can be disposed in the fluid channel 12. In some examples, the actuator 131 can actuate the fluid by one or more of squeezing, pushing, sucking, and pulling. For example, referring to Figure 3C, the actuator 131 can be the piston disposed in the fluid channel 12, which can deliver fluid by pushing. As another example, when the fluid is magnetic, the actuator 131 can be a magnet disposed outside the fluid channel 12, which can deliver fluid by pulling.

In some examples, referring to Figure 3A, the actuator 131 can act on the reservoir 122. In some examples, the actuator 131 can actuate the reservoir 122. In some examples, the reservoir 122 can deform under the action of the actuator 131. In some examples, the actuator 131 can deform the reservoir 122 to decrease or increase its volume.

In some examples, actuating the reservoir 122 may mean that the reservoir 122 is deformed or in the process of deformation. In some examples, stopping actuating the reservoir 122 may mean that the reservoir 122 no longer deforms or is not in the process of deformation. For example, actuating the reservoir 122 may mean that the actuator 131 gradually squeezes or pulls the reservoir 122, and stopping actuating the reservoir 122 may mean that the actuator 131 no longer continues to squeeze or pull the reservoir 122. As another example, actuating the reservoir 122 may mean that in the process that the actuator 131 moves away from the reservoir 122, the reservoir 122 gradually returns to its original shape due to the properties of its material, and stopping actuating the reservoir 122 may mean that the reservoir 122 has returned to its original shape.

In some examples, referring to Figure 3A or Figure 3B, the actuator 131 can move toward or away from the reservoir 122. In some examples, during the movement of the actuator 131 toward or away from the reservoir 122, the reservoir 122 can deform, that is, the volume of the reservoir 122 can change.

In some examples, referring to Figure 3A, the volume of the reservoir 122 can decrease by that the reservoir 122 concaves inward. For example, the actuator 131 can penetrate the reservoir 122 to cause the reservoir 122 to concave inward.

In some examples, referring to Figure 3B, the volume of the reservoir 122 can increase by the reservoir 122 expanding outward. For example, the reservoir 122 returns to its original shape after concavation.

In some examples, the reservoir 122 returning to its original shape can mean that the reservoir 122 returns to its factory condition. In some examples, the reservoir 122 can return to its original shape through its own action, such as through the elasticity of its material. In some examples, the reservoir 122 can also return to its original shape through the action of the actuator 131. For example, the actuator 131 can be connected to the reservoir 122, and when the actuator 131 moves away from the reservoir 122, it can cause the recessed portion of the reservoir 122 to expand outward to restore its original shape.

In some examples, referring to Figure 3A, the actuator 131 can deform the reservoir 122 through direct contact. For example, the actuator 131 can squeeze or pull the reservoir 122 to deform it.

In other examples, referring to Figure 3D, the actuator 131 can deform the reservoir 122 in a non-contact manner. For example, the actuator 131 and the reservoir 122 can be made of magnetic material, thereby enabling the actuator 131 to deform the reservoir 122 without contacting it, based on the principle of like poles repelling or unlike poles attracting.

In some examples, the reservoir 122 can be deformed with a preset deformation amount. In some examples, when the reservoir 122 deforms to the preset deformation amount, the reservoir 122 can provide or receive a preset volume of fluid. That is, the preset deformation amount can be equal to the preset volume.

As described above, the actuator 131 can penetrate the reservoir 122. In some examples, the penetration amount of the actuator 131 can be equal to the deformation amount of the reservoir 122. In this case, by adjusting the penetration amount of the actuator 131, the volume of fluid provided by the reservoir 122 can be adjusted.

In some examples, when the actuator 131 actuates the reservoir 122 to deform with the preset deformation amount, the actuator 131 can be located at a first preset position (see Figure 3A); when the reservoir 122 returns to its original state, the actuator 131 can be located at a second preset position (see Figure 3B). That is, the first preset position can be the position of the actuator 131 when the reservoir 122 deforms with the preset deformation amount, and the second preset position can be the position of the actuator 131 when the reservoir 122 returns to its original state.

In some examples, during the process of moving from the first preset position to the second preset position or moving from the second preset position to the first preset position, the moving distance of the actuator 131 can be a preset distance. In some examples, after moving the preset distance, the actuator 131 can move from the first preset position to the second preset position or move from the second preset position to the first preset position. In some examples, the movement of the actuator 131 can be translation or rotation.

It should be noted that since there can be multiple second preset positions, there can also be multiple preset distances. The size of the preset distance can be set according to requirements. For example, a larger preset distance can be set so that the actuator 131 is far away from the reservoir 122 when in the second preset position, thereby preventing the actuator 131 from accidentally actuating the reservoir 122. Furthermore, for ease of description, it can be assumed that the actuator 131 can move from the first preset position to the second preset position or from the second preset position to the first preset position after moving the preset distance. The preset angle described later is similar and will not be repeated here.

In some examples, the actuator 131 can be made of at least one of stainless steel, aluminum alloy, polyamide, polyetheretherketone, and titanium alloy. Thus, it improves the corrosion resistance and durability of the actuator 131.

In some examples, the thickness of the actuator 131 can be 1 mm to 2 mm. For example, the thickness of the actuator 131 can be 1 mm, 1.1 mm, 1.2 mm, 1.3 mm, 1.4 mm, 1.5 mm, 1.6 mm, 1.7 mm, 1.8 mm, 1.9 mm, or 2 mm, etc. Preferably, the thickness of the actuator 131 can be 1.5 mm.

In some examples, the thickness of the actuator 131 can be the width of the contact surface between the actuator 131 and the reservoir 122. In some examples, the thickness of the actuator 131 can be greater than or equal to the outer diameter of the reservoir 122. Thus, it allows for complete contact between the reservoir 122 and the actuator 131, thereby improving the actuation effect of the actuator 131.

In some examples, referring to Figure 3A, the actuator 131 can have an actuating portion 1311. In some examples, the actuator 131 can act on the reservoir 122 through the actuating portion 1311. In some examples, the actuating portion 1311 can actuate the reservoir 122. In some examples, at least a part of the actuating portion 1311 can penetrate the reservoir 122.

In some examples, the actuator 131 may have a first end 1312 (see Figure 3A). In some examples, the actuating force can act on the first end 1312 to actuate the actuator 131.

In some examples, the movement of the actuator 131 can be oscillating. In some examples, referring to Figure 3A and Figure 3B, the actuator 131 may have a central fulcrum A. In some examples, the actuator 131 can oscillate around the central fulcrum A. In this case, the torque generated by the actuating force can be increased by increasing the distance between the first end 1312 and the central fulcrum A, thereby reducing the actuating force required to actuate the fluid and helping to extend the service life of the fluid delivery device 1.

In some examples, referring to Figure 3A and Figure 3B, the fluid delivery device 1 may have a central axis CA. In some examples, the central fulcrum A may be located on the central axis CA.

In some examples, the actuator 131 may be fixed to the substrate 15 via the central fulcrum A.

In some examples, the first end 1312 may be the end of the actuator 131 near a first lever 132 (described later).

In some examples, the actuator 131 may have a second end 1313 (see Figure 3A). In some examples, the actuating force may be applied to the second end 1313 to actuate the actuator 131. In some examples, the second end 1313 may be the end of the actuator 131 near a second lever 133 (described later).

In some examples, the central fulcrum A may be located between the first end 1312 and the second end 1313. In some examples, the central fulcrum A, the first end 1312, and the second end 1313 may be located on the same straight line.

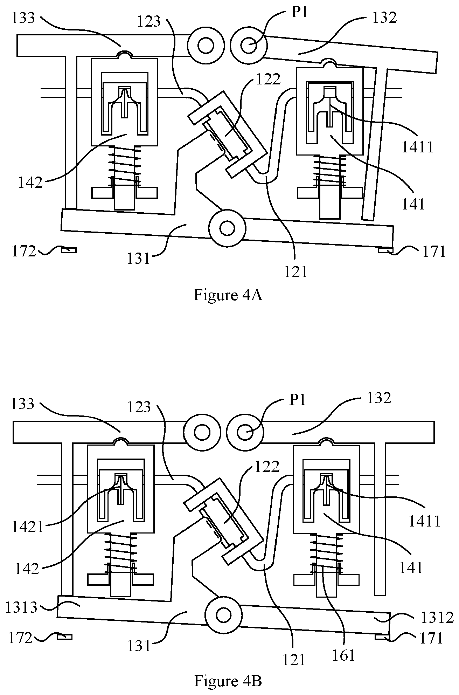

Figure 4A is a schematic diagram illustrating the first lever 132 located at a third preset position according to an example of this disclosure. Figure 4B is a schematic diagram illustrating the first lever 132 located at a fourth preset position according to an example of this disclosure.

In some examples, the actuation component 13 may include the first lever 132 (see Figure 4A or Figure 4B). In some examples, the first lever 132 may be configured to transmit the actuating force. In some examples, the first lever 132 may receive the actuating force. In some examples, the first lever 132 may provide the actuating force.

In some examples, referring to Figure 4A, the first lever 132 can act on the actuator 131. In some examples, the first lever 132 can transmit the actuating force to the actuator 131. In some examples, the first lever 132 can provide the actuating force to the actuator 131. In some examples, the first lever 132 can actuate the actuator 131. In some examples, the first lever 132 can actuate the actuator 131 to move towards the reservoir 122. In some examples, the first lever 132 can actuate the fluid in the reservoir 122 by actuating the actuator 131. In some examples, the actuation manner can be pushing or pulling.

In some examples, the first lever 132 can directly act on the fluid in the reservoir 122.

In some examples, referring to Figure 2, the first lever 132 can be movably disposed on the substrate 15. In some examples, the first lever 132 can be a pendulum lever, that is, the movement of the first lever 132 can be oscillating. In some examples, the first lever 132 can have a first fulcrum P1 (see Figure 4A or Figure 4B). In some examples, the first lever 132 can oscillate around the first fulcrum P1. In some examples, the first lever 132 can be fixed to the substrate 15 via the first fulcrum P1.

In some examples, referring to Figure 4A, when the first lever 132 oscillates to move the actuator 131 a preset distance, the first lever 132 can be located at the third preset position; referring to Figure 4B, when the first lever 132 resets, the first lever 132 can be located at the fourth preset position. In some examples, during the movement from the third preset position to the fourth preset position or from the fourth preset position to the third preset position, the oscillation angle of the first lever 132 can be a preset angle.

In some examples, the third preset position can be the position of the first lever 132 after it pushes the actuator 131 to move the actuator 131 a preset distance, and the fourth preset position can be the position of the first lever 132 after it resets.

In some examples, the first lever 132 actuating the actuator 131 to move the actuator 131 a preset distance can mean that the first lever 132 and a first holding member 171 (described later) jointly actuate the actuator 131 to move the preset distance, or that the first lever 132 actuates the actuator 131 to move the preset distance.

In some examples, the joint actuation of the actuator 131 by the first lever 132 and the first holding member 171 to move the preset distance can mean that the actuator 131 is firstly actuated to move a certain distance by the first lever 132, and then actuated to move a certain distance by the first holding member 171. The sum of the distances actuated by the first lever 132 and the first holding member 171 is the preset distance. In this case, when the first lever 132 is at the third preset position, the actuator 131 is not at the first preset position.

In some examples, the actuation of the actuator 131 by the first lever 132 to move a preset distance can mean that the actuator 131 is actuated to move the preset distance by only the first lever 132. In this case, when the first lever 132 is at the third preset position, the actuator 131 is at the first preset position.

For ease of description, it can be assumed that after the first lever 132 swings a preset angle, the actuator 131 can move a preset distance.

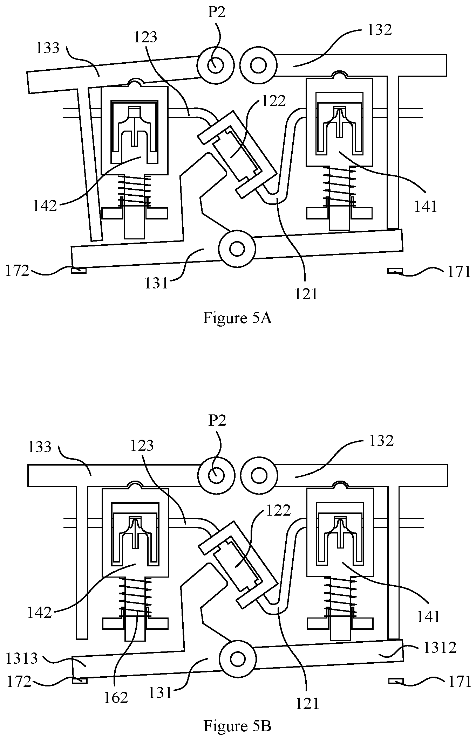

Figure 5A is a schematic diagram illustrating a second lever 133 located at the third preset position according to an example of this disclosure. Figure 5B is a schematic diagram illustrating the second lever 133 located at the fourth preset position according to an example of this disclosure.

In some examples, referring to Figure 5A or Figure 5B, the actuation component 13 may include the second lever 133. The description of the second lever 133, the second fulcrum P2, and the principle of the second lever 133 actuating the actuator 131 can be referred to the relevant description of the first lever 132. The following description only focuses on the differences between the second lever 133 and the first lever 132; identical or similar content will not be repeated.

In some examples, the second lever 133 can actuate the actuator 131 to move away from the reservoir 122. In some examples, the second lever 133 can actuate the actuator 131 to actuate the fluid in the first channel 121 and/or the second channel 123.

In some examples, the second lever 133 can directly act on the fluid in the first channel 121 and/or the second channel 123.

In some examples, referring to Figure 5A or Figure 5B, the second lever 133 can have the second fulcrum P2. In some examples, the second lever 133 can oscillate around the second fulcrum P2. In some examples, the second lever 133 can be fixed to the substrate 15 via the second fulcrum P2.

In some examples, referring to Figure 2, the actuation component 13 can include a power source 134. Thus, it enables the actuation component 13 to provide the actuating force.

In some examples, the power source 134 can be configured to provide the actuating force. In some examples, the power source 134 can provide the actuating force to the actuator 131. In some examples, the power source 134 can also provide the actuating force to the first lever 132. In some examples, the power source 134 can also provide the actuating force to the second lever 133. The following description uses the example of the power source 134 providing power to the first lever 132.

In some examples, referring to Figure 2, the power source 134 may include a first power component 1341.

In some examples, the first power component 1341 may be configured to actuate the first lever 132. In some examples, the first power component 1341 may act on the first lever 132. In some examples, the first power component 1341 may provide the actuating force to the first lever 132. In some examples, the first lever 132 may oscillate under the action of the first power component 1341. In some examples, the first power component 1341 may be connected to the first lever 132.

In some examples, the first power component 1341 may exert the actuating force on the actuator 131 through the first lever 132.

In some examples, the first power component 1341 may also be configured to actuate a first valve 141 (described later).

In some examples, the first power component 1341 may be a memory metal drive device. In some examples, at least a part of the first power component 1341 may be made of shape memory metal material.

In some examples, the shape memory metal material can expand or contract upon its own temperature changes. In some examples, the shape memory metal material may be a nickel-titanium alloy.

The following describes the shape memory metal material is a nickel-titanium alloy, meaning that increasing the temperature of the shape memory metal material can cause it to contract, and decreasing the temperature of the shape memory metal material can cause it to expand.

In some examples, the first power component 1341 may include a first shape memory metal wire, a first power source, and a first controller.

In some examples, the first shape memory metal wire may be made of the shape memory metal material.

In some examples, the length of the first memory metal wire can be from 20 mm to 40 mm. For example, the length of the first memory metal wire can be 20 mm, 25 mm, 30 mm, 35 mm, or 40 mm, etc. Preferably, the length of the first memory metal wire can be 30 mm.

In some examples, the first power source can provide electrical energy to the first memory metal wire.

In some examples, the first controller can control the shape change of the first memory metal wire. Specifically, since the temperature of the first memory metal wire changes according to the duration of the electrical power being on or off, as well as the magnitude of the current flowing through the first memory metal wire when energized, the first controller can control the first power source to control the time of power on or off of the first memory metal wire and the magnitude of the current flowing through it, thereby controlling the shape change of the first memory metal wire. For example, the power on time can be extended or the current can be increased, thereby causing the first memory metal wire to heat up and contract.

In some examples, the first power component 1341 can actuate the first lever 132 through the first memory metal wire. In some examples, the first memory metal wire can act on the first lever 132. In some examples, the first memory metal wire can be connected to the first lever 132. In some examples, the first memory metal wire can actuate the first lever 132 by its own contraction or extension.

In some examples, the first power component 1341 may also include a servo motor, an electromagnetic drive mechanism, or other devices that can provide power.

In some examples, referring to Figure 2, the power source 134 may include a pulley 1343. In some examples, the first memory metal wire can be wound around the pulley 1343. When the length of the first memory metal wire meets the actuation requirements, the space utilization of the first memory metal wire can be improved through the pulley 1343 structure, thereby making the structure of the first power component 1341 more compact and facilitating the miniaturization of the fluid delivery device 1.

As described above, one surface of the substrate 15 can be adhered to the body surface of the target 2. In some examples, the first memory metal wire can be disposed on the surface of the substrate 15 away from the body surface of the target 2. In this case, since the degree of contraction of the first memory metal wire is temperature-dependent, the influence of the temperature of the target 2 on the first memory metal wire can be reduced, thereby facilitating the normal operation of the fluid delivery device 1.

In some examples, referring to Figure 2, the power source 134 may include a second power component 1342. The structure, material, and cooperation with the second lever 133 of the second power component 1342 can be described with reference to the relevant description of the first power component 1341, and will not be repeated here.

In some examples, the second power component 1342 may include a second memory metal wire, a second power supply, and a second controller. The interaction principle between the second memory metal wire, the second power supply and the second controller, as well as the material and size of the second memory metal wire, can be described with reference to the relevant descriptions of the first power supply, the first controller, and the first memory metal wire, and will not be repeated here.

As described above, referring to Figure 2, the fluid delivery device 1 may include a flow limiting component 14 that can restrict fluid flow. In some examples, restricting fluid flow may refer to restricting at least one of the fluid flow rate, flow velocity, and flow direction. In some examples, the flow limiting component 14 can restrict the flow of fluid in the fluid channel 12. In some examples, the flow limiting component 14 can squeeze the fluid channel 12 to restrict the flow of fluid.

In some examples, the flow limiting component 14 can be configured to open or close the first channel 121 and/or the second channel 123. In this case, controlling the opening and closing of the first channel 121 and the second channel 123 by the flow limiting component 14 can help to make the fluid flow along a desired path.

In some examples, the flow limiting component 14 can restrict the flow of fluid in the first channel 121. In some examples, the flow limiting component 14 can also restrict the flow of fluid in the second channel 123.

In some examples, in response to the flow limiting component 14 opening the first channel 121 and/or the second channel 123, the actuation component 13 can provide the actuating force. In this case, the reservoir 122 is first connected to the first channel 121 and/or the second channel 123, and then the actuating force is provided via the actuation component 13, thereby improving the utilization rate of the actuating force.

In some examples, the flow limiting component 14 may include a valve disposed on the fluid flow path in the first channel 121 and/or the second channel 123. In some examples, the flow limiting component 14 may act on the first channel 121 and/or the second channel 123 to close or open the fluid flow path.

In some examples, referring to Figure 2, the flow limiting component 14 may include a first valve 141. In some examples, the first valve 141 may be disposed on the fluid outflow side of the fluid delivery device 1. In some examples, the first valve 141 may be disposed on the fluid flow path in the first channel 121.

In some examples, the first valve 141 may be configured to control the fluid flow within the first channel 121.

In some examples, the first valve 141 may switch states in response to actuation of the first power component 1341. In some examples, the first valve 141 may also switch states in response to the push of the first lever 132. In some examples, the first valve 141 may also switch states in response to fluid flow.

In some examples, the first valve 141 may be an active valve or a passive valve.

In some examples, the first valve 141 may be any one of a one-way valve and a two-way valve.

In some examples, the first valve 141 may be at least one of a gate valve, a check valve, a non-return valve, a globe valve, a ball valve, and a diaphragm valve. Preferably, the first valve 141 may be a gate valve.

In some examples, the first valve 141 may include both an open and a closed state, that is, the first valve 141 may have an open state and a closed state. In some examples, the open state may refer to the state of the first valve 141 when fluid can flow into or out of the reservoir 122 via the first channel 121. In some examples, the closed state may refer to the state of the first valve 141 when fluid cannot flow into or out of the reservoir 122 via the first channel 121.

In some examples, referring to Figure 2, the first valve 141 may include a first opening and closing member 1411 which can be configured to control the opening or closing of the first valve 141.

In some examples, referring to Figure 4A, when the first valve 141 is at the open state, the first opening and closing member 1411 may be located at a fifth preset position; referring to Figure 4B, when the first valve 141 is at the closed state, the first opening and closing member 1411 may be located at a sixth preset position. In some examples, the first opening and closing member 1411 can move from the fifth preset position to the sixth preset position to switch the first valve 141 from the open state to the closed state; the first opening and closing member 1411 can move from the sixth preset position to the fifth preset position to switch the first valve 141 from the closed state to the open state. In some examples, the fifth preset position can be the position of the first opening and closing member 1411 after the first valve 141 is opened, and the sixth preset position can be the position of the first opening and closing member 1411 after the first valve 141 is closed.

In some examples, since the first opening and closing member 1411 can continue to move after the first valve 141 is opened, there can be multiple fifth preset positions.

In some examples, when the first opening and closing member 1411 is at the fifth preset position, the first opening and closing member 1411 may not compress the first channel 121, and the first channel 121 can return to its original state. Thus, the first valve 141 can be opened.

In some examples, when the first opening and closing member 1411 is at the sixth preset position, the first opening and closing member 1411 can compress the first channel 121 to deform it. In some examples, under the compression of the first opening and closing member 1411, the inner walls of the first channel 121 can adhere to each other to seal the internal space. Thus, the first valve 141 can be closed.

In some examples, during the process of the first opening and closing member 1411 moving from the fifth preset position to the sixth preset position, the first channel 121 can be gradually compressed. In this case, by controlling the movement distance of the first opening and closing member 1411, the degree of compression of the first channel 121 can be controlled, thereby controlling the degree of restriction of the fluid in the first channel 121 by the first valve 141.

In some examples, the first valve 141 can be a knife gate valve, and the first opening and closing member 1411 can be the gate of the knife gate valve. In some examples, the height of the gate of the knife gate valve can be 2 mm to 4 mm. For example, it can be 2 mm, 2.5 mm, 3 mm, 3.5 mm, or 4 mm. Preferably, the height of the gate of the knife gate valve can be 3 mm. In some examples, the first opening and closing member 1411 can be made of at least one of carbon steel and stainless steel.

In some examples, referring to Figure 2, the first valve 141 can have a first engaging portion 1412. In some examples, the first engaging portion 1412 can be provided on the first valve 141. In some examples, the first lever 132 can have a first mating portion 1321. In some examples, the first engaging portion 1412 may mate with the first mating portion 1321. In some examples, the contour of the first engaging portion 1412 may be similar to or the same as that of the first mating portion 1321.

In some examples, referring to Figure 2, the contours of the first engaging portion 1412 and the first mating portion 1321 may be arc-shaped. In this case, since the direction of the force exerted by the first lever 132 on the first opening and closing member 1411 changes with the swing of the first lever 132, the arc-shaped structure of the first engaging portion 1412 and the first mating portion 1321 can increase the component force exerted by the first lever 132 in the direction of movement of the first opening and closing member 1411, thereby helping to reduce energy consumption.

In some examples, referring to Figure 2, the flow limiting component 14 may include a second valve 142. In some examples, the second valve 142 may be disposed on the side of the fluid inflow of the fluid delivery device 1. In some examples, the second valve 142 can be disposed on the fluid flow path in the second channel 123. The working principle of the second valve 142 and the second valve can be referred to the relevant description of the first valve 141, and will not be repeated here.

In some examples, both the first valve 141 and the second valve 142 can be active valves or both can be passive valves. In some examples, the first valve 141 and the second valve 142 can also be an active valve and a passive valve (or a passive valve and an active valve), respectively.

In some examples, the first valve 141 and the second valve 142 can be respectively disposed on two ends of the reservoir 122 (see Figure 2). In some examples, the first valve 141 and the second valve 142 can be disposed in the first channel 121 and the second channel 123, respectively. In some examples, the first valve 141 can be disposed at any position in the first channel 121, for example, located in the first channel 121 near the reservoir 122. The second valve 142 can be disposed at any position in the second channel 123, for example, located in the second channel 123 near the reservoir 122.

As described above, the first channel 121 can be connected to the reservoir 122. In some examples, the first channel 121 can be connected to the reservoir 122 via the first valve 141. As described above, the second channel 123 can be connected to the reservoir 122. In some examples, the second channel 123 can be connected to the reservoir 122 via the second valve 142.

In some examples, the second valve 142 may include a second opening and closing member 1421 which can be configured to control opening or closing of the second valve 142. The principle of the second opening and closing member 1421 controlling opening or closing of the second valve 142 can be referred to the relevant description of the first opening and closing member 1411, and will not be repeated here.

In some examples, the second valve 142 may have a second engaging portion 1422. In some examples, the second lever 133 may have a second mating portion 1331. The mating relationship and working principle of the second engaging portion 1422 and the second mating portion 1331 can be referred to the relevant description of the first engaging portion 1412 and the first mating portion 1321, and will not be repeated here.

In other examples, when the first valve 141 does not switch states in response to the push of the first lever 132, the first valve 141 may not have the first engaging portion 1412, and the first lever 132 may not have the first mating portion 1321. The second valve 142 may do the same.

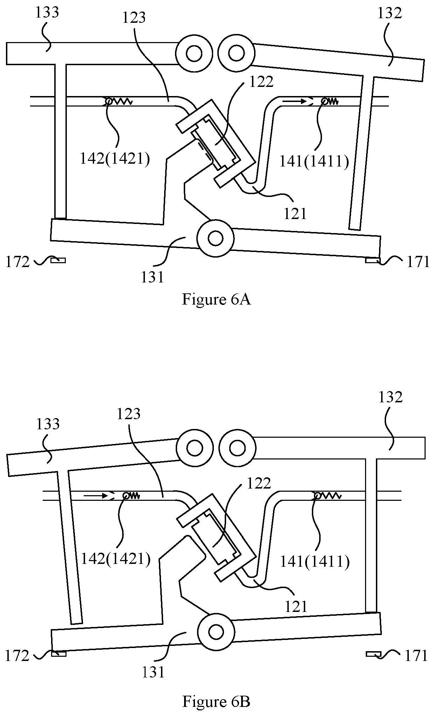

Figure 6A is a schematic diagram illustrating the reservoir 122 supplying fluid when the valve is a passive valve according to an example of this disclosure. Figure 6B is a schematic diagram illustrating the reservoir 122 receiving fluid when the valve is the passive valve according to an example of this disclosure.

As described above, the first valve 141 and/or the second valve 142 can also be passive valves. In some examples, the first valve 141 and/or the second valve 142 can be one-way valves.

In some examples, referring to Figure 6A, when the fluid is supplied from the reservoir 122, the pressure of the fluid in the reservoir 122 increases, thereby opening the first valve 141, moving the first opening and closing member 1411 to the fifth preset position, while simultaneously inhibiting opening of the second valve 142. The second opening and closing member 1421 is held in the sixth preset position.

In some examples, referring to Figure 6B, when the reservoir 122 receives the fluid, the pressure of the fluid in the reservoir 122 decreases, which can open the second valve 142, move the second opening and closing member 1421 to the fifth preset position, while simultaneously inhibiting opening of the first valve 141. The first opening and closing member 1411 is held in the sixth preset position.

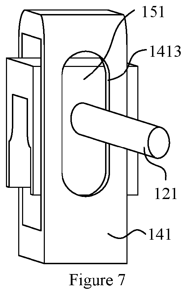

Figure 7 is a schematic diagram illustrating a first position-limiting portion 1413 according to an example of this disclosure.

In some examples, referring to Figure 7, the first valve 141 may have the first position-limiting portion 1413.

In some examples, the first position-limiting portion 1413 may be configured to limit the position of the first valve 141. In some examples, the first position-limiting portion 1413 may be provided on the first opening and closing member 1411. In some examples, the size of the first position-limiting portion 1413 may be equal to or slightly larger than the size of the first channel 121.

In some examples, the first position-limiting portion 1413 may be a guide rail structure. In some examples, the first position-limiting portion 1413 may extend along the moving direction of the first opening and closing member 1411. In some examples, the first opening and closing member 1411 may move along a path limited by the first position-limiting portion 1413.

In some examples, the first channel 121 may pass through the first position-limiting portion 1413. In some examples, the first opening and closing member 1411 may move relative to the first channel 121 and along a guide path of the first position-limiting portion 1413.

In some examples, the second valve 142 may have a second position-limiting portion. In some examples, the second position-limiting portion may be configured to limit the position of the second valve 142. The related description of the second position-limiting portion can be referred to the relevant description of the first position-limiting portion 1413, and will not be repeated here.

In some examples, a first power component 1341 can actuate the first opening and closing member 1411 to move it from the sixth preset position to the fifth preset position (i.e., the first valve 141 opens). In some examples, the first power component 1341 can actuate the actuator 131 to move the actuator 131 from the second preset position to the first preset position.

In some examples, the first power component 1341, the first valve 141, and the actuator 131 can be linked. In some examples, the first power component 1341 can open the first valve 141 and make the actuator 131 act on the reservoir 122. Specifically, in response to the first power component 1341 providing the actuating force, the first valve 141 can open, and the actuator 131 can act on the reservoir 122.

In some examples, during the process of the first power component 1341 providing the actuating force, the first opening and closing member 1411 can move from the sixth preset position to the fifth preset position, and the actuator 131 can move from the second preset position to the first preset position. That is, after providing the actuating force, the first power component 1341 can open the first valve 141, and simultaneously actuate the actuator 131 to move the actuator 131 a preset distance and squeeze the reservoir 122, ultimately causing the reservoir 122 to deform with a preset deformation. In this case, actuation of the first power component 1341 enables the reservoir 122 to supply a predetermined volume of the fluid.

In some examples, the first lever 132 can transmit the actuating force provided by the first power component 1341. In this case, by forming a lever structure with the first lever 132, the torque generated by the actuating force can be increased by increasing the lever arm, thereby reducing the energy consumption of the fluid delivery device 1 and extending its service life.

In some examples, referring to Figure 4A, the first power component 1341 can actuate the first lever 132 to move it from the fourth preset position to the third preset position. In some examples, the first lever 132 can actuate the first opening and closing member 1411 to move it from the sixth preset position to the fifth preset position. In some examples, the first lever 132 can actuate the actuator 131 to move the actuator 131 from the second preset position to the first preset position.

In some examples, the first power component 1341, the first lever 132, the first valve 141, and the actuator 131 can be linked. In some examples, the first power component 1341 can actuate the first lever 132, thereby opening the first valve 141 and making the actuator 131 act on the reservoir 122. Specifically, in response to the actuating force provided by the first power component 1341, the first lever 132 can move from the fourth preset position to the third preset position, and during the movement of the first lever 132, the first valve 141 can be opened, and the actuator 131 can act on the reservoir 122.

In some examples, during the movement of the first lever 132 from the fourth preset position to the third preset position, the first opening and closing member 1411 can move from the sixth preset position to the fifth preset position, and the actuator 131 can move from the second preset position to the first preset position. That is, after swinging a preset angle, the first lever 132 can push and open the first valve 141, and simultaneously push the actuator 131 to move the actuator 131 a preset distance and squeeze the reservoir 122, which causes the reservoir 122 to deform with a preset amount. In this case, the actuation of the first power component 1341 enables the reservoir 122 to provide a preset volume of the fluid.

It should be noted that due to the characteristics of the shape memory metal material, the actuating force gradually decreases, which in turn causes the swing angle of the first lever 132 to gradually decrease. Therefore, as the fluid delivery device 1 is used, the third preset position can change. To ensure that the first lever 132 can open the first valve 141, the preset angle can be set to a relatively large value, which helps to avoid the situation where the first valve 141 cannot be opened due to the swing angle of the first lever 132 being too small. The same applies to the second lever 133 and the second valve 142.

In some examples, the second power component 1342 can actuate the second opening and closing member 1421 to move it from the sixth preset position to the fifth preset position (i.e., the second valve 142 opens). In some examples, the second power component 1342 can actuate the actuator 131 to move the actuator 131 from the first preset position to the second preset position.

In some examples, the second power component 1342, the second valve 142, and the actuator 131 can be linked. In some examples, the second power component 1342 can open the second valve 142 and restore the reservoir 122 to its original state. Specifically, in response to the second power component 1342 providing the actuating force, the second valve 142 can open, and the reservoir 122 can return to its original state.

In some examples, during the process of the second power component 1342 providing the actuating force, the second opening and closing member 1421 can move from the sixth preset position to the fifth preset position, and the actuator 131 can move from the first preset position to the second preset position. That is, after providing the actuating force, the second power component 1342 can open the second valve 142, and simultaneously actuate the actuator 131 to move the actuator 131 a preset distance away from the reservoir 122, ultimately restoring the reservoir 122 to its original state. In this case, the actuation of the second power component 1342 enables the reservoir 122 to receive a preset volume of fluid.

In some examples, the second lever 133 can transmit the actuating force provided by the second power component 1342. In this case, by forming a lever structure with the second lever 133, the torque generated by the actuating force can be increased by increasing the lever arm, thereby reducing the energy consumption of the fluid delivery device 1 and extending its service life.

In some examples, referring to Figure 5A, the second power component 1342 can actuate the second lever 133 to move it from the fourth preset position to the third preset position. In some examples, the second lever 133 can actuate the second opening and closing member 1421 to move it from the sixth preset position to the fifth preset position. In some examples, the second lever 133 can actuate the actuator 131 to move the actuator 131 from the first preset position to the second preset position.

In some examples, the second power component 1342, the second lever 133, the second valve 142, and the actuator 131 can be linked. In some examples, the second power component 1342 can actuate the second lever 133 to open the second valve 142 and restore the reservoir 122 to its original state. Specifically, in response to the actuating force provided by the second power component 1342, the second lever 133 can move from the fourth preset position to the third preset position, and during the movement of the second lever 133, the second valve 142 can open, and the reservoir 122 can return to its original state.

In some examples, during the movement of the second lever 133 from the fourth preset position to the third preset position, the second opening and closing member 1421 can move from the sixth preset position to the fifth preset position, and the actuator 131 can move from the first preset position to the second preset position. That is, after swinging a preset angle, the second lever 133 can push and open the second valve 142, and simultaneously push the actuator 131 to move the actuator 131 a preset distance away from the reservoir 122, so that the reservoir 122 returns to its original state. In this case, the actuation of the second power component 1342 enables the reservoir 122 to receive a preset volume of fluid.

It should be noted that when the fluid delivery device 1 only has the first channel 121, or only has the first channel 121 and the reservoir 122, the actuation component 13 may not have the second lever 133 and the second power component 1342, and the flow limiting component 14 may not have the second valve 142.

In some examples, the flow limiting component 14 can alternately open the first channel 121 and the second channel 123. That is, when the first channel 121 is open, the second channel 123 can be closed; when the second channel 123 is open, the first channel 121 can be closed. In this case, by alternately opening the first channel 121 and the second channel 123, the fluid can flow into or out of the reservoir 122 through one of the first channel 121 and the second channel 123, thereby enabling directional fluid flow.

In some examples, referring to Figure 2, the fluid delivery device 1 may include a reset component 16. In some examples, the reset component 16 may be configured to reset the flow limiting component 14. In some examples, the reset component 16 may push or pull the flow limiting component 14.

In some examples, referring to Figure 2, the reset component 16 may include a first reset member 161.

In some examples, the first reset member 161 may be configured to switch the state of the first valve 141. In some examples, the first valve 141 may switch its state in response to actuation of the first reset member 161. In some examples, the first reset member 161 may actuate the first valve 141 in response to the first power component 1341 ceasing to provide the actuating force.

In some examples, referring to Figure 4B, the first reset member 161 may close the first valve 141. In some examples, the first reset member 161 may be disposed on the first valve 141.

In some examples, the first valve 141 and the first lever 132 may be linked. In some examples, after the first power component 1341 ceases to provide the actuating force, the first reset member 161 may actuate the first valve 141 to close the first valve 141. In some examples, the first reset member 161 may actuate the first lever 132 to move it from the third preset position to the fourth preset position via the first valve 141 so as to reset the first lever 132.

In some examples, the first reset member 161 can actuate the first opening and closing member 1411 to move it from the fifth preset position to the sixth preset position (i.e., the first valve 141 closes).

In some examples, one end of the first reset member 161 can be connected to the first opening and closing member 1411. In some examples, the other end of the first reset member 161 (i.e., the end not connected to the first opening and closing member 1411) can be fixed to the substrate 15.

In some examples, the first power component 1341 can actuate the first lever 132 to move it from the third preset position to the fourth preset position. Specifically, the first power component 1341 can extend after power is cut off, thereby actuating the first lever 132 to move it from the third preset position to the fourth preset position.

In some examples, the first reset member 161 can be one of a coil spring, a suspension spring, or a compression spring. In some examples, the first reset member 161 can also be a servo motor or other power-providing device.

In some examples, the length of the first reset member 161 can be 2 mm to 4 mm. For example, it can be 2 mm, 2.5 mm, 3 mm, 3.5 mm, or 4 mm. Preferably, the length of the first reset member 161 can be 3 mm.

In some examples, the reset component 16 may include a second reset member 162 (see Figure 5B). In some examples, the second reset member 162 can be configured to switch the state of the second valve 142. The structure and size of the second reset member 162, and the interaction between the second reset member 162, the second valve 142, and the second power component 1342, can be referred to the related description of the first reset member 161, and will not be repeated here.

As described above, the flow limiting component 14 can alternately open the first channel 121 and the second channel 123. Specifically, after the first reset member 161 closes the first valve 141, the second lever 133 can open the second valve 142 and actuate the actuator 131 to move to the second preset position so that the reservoir 122 receives fluid; after the reservoir 122 receives a preset volume of fluid, the second reset member 162 can close the second valve 142, and after the second valve 142 is closed, the first lever 132 can open the first valve 141 and actuate the actuator 131 to move to the first preset position so that the reservoir 122 provides fluid; after the reservoir 122 provides a preset volume of fluid, the first reset member 161 can close the first valve 141.

In some examples, referring to Figure 2, the fluid delivery device 1 may include a holding component 17. In some examples, the holding component 17 may be configured to hold the position of the actuator 131.

In some examples, referring to Figure 4B or Figure 5B, the holding component 17 may hold the actuator 131 in the first preset position or the second preset position. Thus, it enables the reservoir 122 to provide or receive a predetermined volume of fluid.

In some examples, the holding component 17 may be detachably or releasably connected to the actuator 131. In some examples, the holding component 17 may be connected to the actuator 131 by one or more of the following methods: attraction, adhesion, snap-fit, and friction connection. For example, magnets may be provided on the holding component 17 and the actuator 131 to attract them together; for another example, an adhesive layer may be provided between the holding component 17 and the actuator 131 to adhere them; for another example, the holding component 17 and the actuator 131 may be snap-fitted by at least one of the following structures: latch, hook, bolt, or pin; for another example, the holding component 17 and the actuator 131 may be a mating structure and may be held together by friction between their surfaces.

In some examples, the holding component 17 may be configured to provide the actuating force. In some examples, the holding component 17 may act on the actuator 131. In some examples, the holding component 17 can provide the actuating force to the actuator 131.

In some examples, the holding component 17 can actuate the actuator 131 to move a preset distance. In some examples, the holding component 17 can actuate the actuator 131 together with the first lever 132 or the second lever 133. In this case, since the shape memory metal material gradually fatigues with increasing use, resulting in a smaller actuating force provided by the power source 134, providing the actuating force by the holding component 17 can reduce the requirement for the actuating force generated by the shape memory metal material and reduce the energy consumption of the fluid delivery device 1.

In some examples, the holding component 17 may include a first holding member 171. In some examples, the first holding member 171 can be configured to hold the actuator 131 in the first preset position. In some examples, the first holding member 171 can hold the position of the first end 1312. In some examples, when the first end 1312 approaches or contacts the first holding member 171, the first holding member 171 can connect to the first end 1312 to maintain its position. In some examples, when the first end 1312 is connected to the first holding member 171, the actuator 131 can be located at the first preset position.

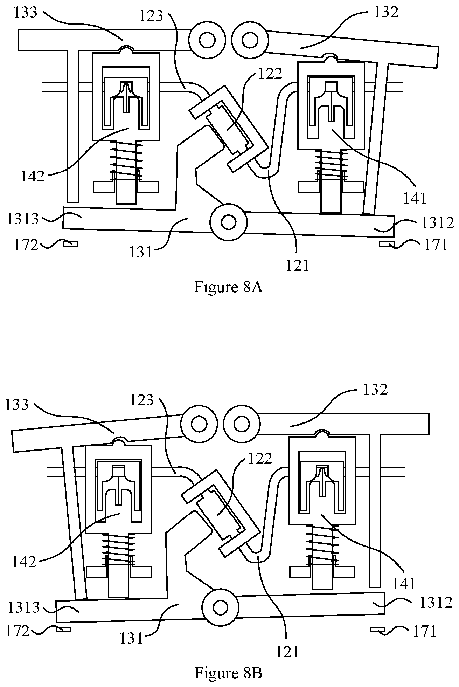

Figure 8A is a schematic diagram illustrating the first holding member 171 actuating the first end 1312 according to an example of this disclosure. Figure 8B is a schematic diagram illustrating the second holding member 172 actuating the second end 1313 according to an example of this disclosure.

In some examples, the first holding member 171 can be configured to actuate the first end 1312.

In some examples, when the first end 1312 approaches the first holding member 171 (e.g., see Figure 8A, when the first lever 132 pushes the first end 1312 towards the first holding member 171), the first holding member 171 can provide an actuating force to the first end 1312.

In some examples, referring to Figures 8A and 4A, under the action of the first holding member 171, the first end 1312 can move toward the first holding member 171 until it connects with the first holding member 171. Specifically, the first end 1312 can move from the position in Figure 8A to the position in Figure 4A.

In some examples, the first holding member 171 can be fixed to the substrate 15. Thus, the first holding member 171 can more stably hold the position of the first end 1312 or provide the actuating force.

In some examples, the first holding member 171 can be disposed on the side close to the first end 1312. In some examples, the first holding member 171 may be disposed on the movement path of the first end 1312. In some examples, the first holding member 171 may be disposed above or below the first end 1312. Preferably, the first holding member 171 may be disposed below the first end 1312.

In some examples, the first holding member 171 may be a magnet, and the first end 1312 may be magnetic. Thus, the first holding member 171 can attract the first end 1312. In some examples, the first holding member 171 may be a permanent magnet, for example, at least one of a ferrite magnet, a neodymium iron boron magnet, and a cobalt hard magnet.

In some examples, the actuator 131 may be at least partially made of a ferromagnetic material. In some examples, the first end 1312 may be made of a ferromagnetic material, and the portion of the actuator 131 other than the first end 1312 may be made of a non-ferromagnetic or weakly magnetic material.

In other examples, the actuator 131 may also be made of a non-ferromagnetic or weakly magnetic material. In some examples, the first end 1312 may be coated with a ferromagnetic material to keep it magnetically fixed to the first holding member 171. Specifically, the surface of the first end 1312 facing the first holding member 171 may be coated with a ferromagnetic material.

In some examples, the holding component 17 may include a second holding member 172. In some examples, the second holding member 172 may be configured to hold the actuator 131 in the second preset position. In some examples, the second holding member 172 may be configured to actuate the second end 1313 (see Figures 8B and 5A). The functional relationship between the second holding member 172 and the second end 1313 can be referenced in the related description of the functional relationship between the first holding member 171 and the first end 1312, and will not be repeated here.

In some examples, in response to the first lever 132 providing the actuating force, the first valve 141 can open, the first end 1312 can connect to the first holding member 171, and the second end 1313 can be disconnected from the second holding member 172. In some examples, in response to the first lever 132 ceasing to provide the actuating force, the first valve 141 can close under the action of the first reset member 161, and the first end 1312 can remain connected to the first holding member 171.

In some examples, in response to the second lever 133 providing the actuating force, the second valve 142 can open, the second end 1313 can connect to the second holding member 172, and the first end 1312 can be disconnected from the first holding member 171. In some examples, in response to the second lever 133 ceasing to provide the actuating force, the second valve 142 may close under the action of the second reset member 162, and the second end 1313 may remain connected to the second holding member 172.

In some examples, when the first end 1312 is connected to the first holding member 171, the first end 1312 may be in communication with the first holding member 171. In some examples, after the power source 134 provides the actuating force, whether the first channel 121 is blocked or not can be determined by detecting whether the first end 1312 is in communication with the first holding member 171. Specifically, after the power source 134 provides the actuating force, the actuator 131 can move to the first preset position and make the reservoir 122 provide a preset volume of fluid. If the first channel 121 is blocked, the reservoir 122 will be unable to provide the preset volume of fluid, the reservoir 122 will be unable to continue deforming, and the movement of the actuator 131 will be obstructed. Consequently, the first end 1312 of the actuator 131 will not be able to connect with the first holding member 171. Therefore, the blockage can be determined by checking the connection state.

In some examples, when the second end 1313 is connected to the second holding member 172, the second end 1313 can be in communication with the second holding member 172. In some examples, after the power source 134 provides the actuating force, whether the second channel 123 is blocked or not can be determined by detecting whether the second end 1313 is in communication with the second holding member 172. Specifically, after the power source 134 provides the actuating force, the actuator 131 can move to the second preset position and allow the reservoir 122 to receive a preset volume of fluid. If the second channel 123 is blocked, the reservoir 122 will be unable to receive the preset volume of fluid, and the reservoir 122 will be unable to continue deforming, resulting in hindering the movement of the actuator 131. Consequently, the second end 1313 of the actuator 131 cannot connect with the second holding member 172. Therefore, the blockage can be determined by checking the connection state. It should be noted that the above description only applies to the case where the actuator 131 is connected to the reservoir 122. If the actuator 131 is not connected to the reservoir 122, the actuator 131 will not be obstructed when moving to the second preset position. In this case, since the second channel 123 is outside the target 2, it is also convenient to directly obtain the blockage status of the second channel 123.

In some examples, the first holding member 171 can apply a first holding force to the first end 1312. In some examples, the second holding member 172 can apply a second holding force to the second end 1313. In some examples, the first holding force can be greater than the second holding force. In this case, since the force exerted by the reservoir 122 on the actuator 131 in the first preset position is greater than the force exerted by the reservoir 122 on the actuator 131 in the second preset position, by making the first holding force greater than the second holding force, the first holding member 171 can more stably hold the actuator 131 in the first preset position, thereby helping the reservoir 122 to provide a preset volume of fluid.

In other examples, the first holding force can also be less than or equal to the second holding force.

As described above, the first holding member 171 can be configured to actuate the first end 1312. In some examples, there can be a first magnetic attraction distance between the first end 1312 and the first holding member 171. In some examples, when the relative distance between the first end 1312 and the first holding member 171 is less than or equal to the first magnetic attraction distance, the first end 1312 can approach the first holding member 171 under attraction of the first holding member 171 until it connects with the first holding member 171.

As described above, the first lever 132 and the first holding member 171 can jointly actuate the actuator 131. Specifically, when the first lever 132 moves from the fourth preset position to the third preset position, it can actuate the actuator 131 until the relative distance between the first end 1312 and the first holding member 171 is less than or equal to the first magnetic attraction distance. In this case, by actuating the actuator 131 with the first lever 132 and attracting the first end 1312 with the first holding member 171, the actuator 131 can be moved a preset distance.

As described above, the actuator 131 can be actuated by the first lever 132 alone. Specifically, when the first lever 132 moves from the fourth preset position to the third preset position, it can actuate the actuator 131 until the first end 1312 connects with the first holding member 171.

In some examples, there may be a second magnetic attraction distance between the second end 1313 and the second holding member 172. In some examples, when the relative distance between the second end 1313 and the second holding member 172 is less than or equal to the second magnetic attraction distance, the second end 1313 can approach the second holding member 172 under the attraction of the second holding member 172 until it connects with the second holding member 172. The description of the actuation of the actuator 131 by the second lever 133 and the second holding member 172 can be referred to the related description of the first lever 132 and the first holding member 171, and will not be repeated here.

As described above, the first holding force can be greater than the second holding force. In some examples, the first magnetic attraction distance can be greater than the second magnetic attraction distance.

In some other examples, the first magnetic attraction distance may be less than or equal to the second magnetic attraction distance.

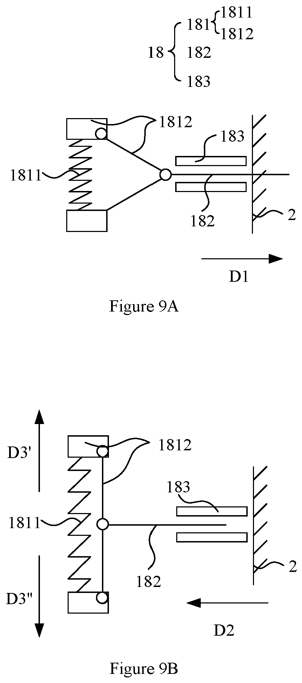

Figure 9A is a schematic diagram illustrating a puncture mechanism 182 entering the target 2 in the first embodiment of the application component 18 according to an example of this disclosure. Figure 9B is a schematic diagram illustrating the puncture mechanism 182 withdrawing from the target 2 in the first embodiment of the application component 18 according to an example of this disclosure.

As described above, the fluid delivery device 1 can apply itself to the body surface of the target 2 through its own structure. In some examples, the fluid delivery device 1 can also apply itself to the body surface of the target 2 through external factors. In some examples, the fluid delivery device 1 can also be applied to the body surface of the target 2 through both its own structure and external factors. In some examples, the external factors can refer to any device or person that can provide driving force.

In some examples, the fluid delivery device 1 may include an application component 18 (see Figures 9A and 9B). In some examples, the application component 18 may be configured to apply at least a portion of a component in the fluid delivery device 1 to the target 2. In some examples, the application component 18 may place at least a portion of the fluid channel 12 subcutaneously into the target 2.

In other examples, the fluid delivery device 1 may not include the application component 18, which may be a component independent of the fluid delivery device 1, i.e., the application component 18 may be an external factor as described above.

In some examples, the application component 18 may include a drive mechanism 181 (see Figures 9A and 9B). In some examples, the drive mechanism 181 may be configured to provide a driving force.

In some examples, the drive mechanism 181 may act on at least a portion of the fluid channel 12 and/or the puncture mechanism 182 (described later).

In some other examples, the application component 18 may not include the drive mechanism 181, and the driving force acting on the fluid channel 12 and/or the puncture mechanism 182 may be provided by external factors. For example, the driving force provided by external factors may be power provided by the device or manpower.

The following description uses the drive mechanism 181 acting on the first channel 121 as an example.

In some examples, the drive mechanism 181 may drive the first channel 121. In some examples, the drive mechanism 181 may drive the first channel 121 to move towards the target 2. In some examples, the drive mechanism 181 may drive the first channel 121 into and be placed subcutaneously in the target 2.

In some examples, the drive mechanism 181 may include a drive source 1811 (see Figures 9A and 9B). In some examples, the drive source 1811 may be configured to provide driving force. In some examples, the number of drive sources 1811 may be one or more.

In some examples, the drive source 1811 can store driving force. In some examples, the driving force of the drive source 1811 can be stored before the fluid delivery device 1 leaves the factory. In some examples, the driving force of the drive source 1811 can also be stored by the user before using the fluid delivery device 1. In some examples, the drive source 1811 can be a spring. For example, it can be a tension spring or a torsion spring.

In other examples, the drive source 1811 can also be a servo motor or other power-providing device.

In some examples, one end of the drive source 1811 can be connected to the first channel 121. In some examples, the other end of the drive source 1811 can be fixed to the substrate 15 or to the housing of the fluid delivery device 1.

In some examples, referring to Figures 9A and 9B, the drive mechanism 181 can include a transmission member 1812. In some examples, the transmission member 1812 can be configured to change the direction of the driving force.

In some examples, the transmission member 1812 may be configured to transmit driving force. In some examples, the transmission member 1812 may receive driving force from the driving source 1811. In some examples, the transmission member 1812 may provide driving force to the first channel 121.

In some examples, the driving source 1811 may be connected to the first channel 121 via the transmission member 1812.

In some examples, the application component 18 may include a puncture mechanism 182 (see Figures 9A and 9B). In some examples, the puncture mechanism 182 may be configured to puncture the target 2 subcutaneously. In some examples, the puncture mechanism 182 may carry at least a portion of the fluid channel 12 into the target 2 subcutaneously. Thus, it facilitates the placement of the fluid channel 12 subcutaneously in the target 2. In some examples, the puncture mechanism 182 may carry the first channel 121 into the target 2 subcutaneously.

In some examples, the direction in which the puncture mechanism 182 penetrates the target 2 subcutaneously can form an angle with the body surface of the target 2. In some examples, the angle can be from 5 degrees to 85 degrees. For example, it can be 30 degrees, 40 degrees, 60 degrees, or 80 degrees. In this case, by making the puncture mechanism 182 penetrate obliquely into the subcutaneous tissue of the target 2, it can help inhibit inflammation or crusting of the target 2.

In other examples, the direction in which the puncture mechanism 182 penetrates the target 2 subcutaneously can also be perpendicular to the body surface of the target 2.

In some examples, the first channel 121 can accommodate the puncture mechanism 182. In some examples, the first channel 121 can be sleeved on the puncture mechanism 182.

In other examples, the first channel 121 can be accommodated within the puncture mechanism 182. In some examples, the puncture mechanism 182 can be sleeved on the first channel 121.

In some other examples, when the first channel 121 is made of a hard material, the fluid delivery device 1 may not include the puncture mechanism 182, and the first channel 121 may penetrate the target 2 subcutaneously due to the hardness of its own material.

In some examples, the puncture mechanism 182 may have a sharp object. In some examples, the first channel 121 may accommodate at least a portion of the sharp object.

In some other examples, the first channel 121 may also be accommodated within the sharp object. In some examples, the sharp object may have a receiving groove extending along the length of the sharp object. In some examples, at least a portion of the first channel 121 may be accommodated within the receiving groove.

In some examples, the drive mechanism 181 may act on the puncture mechanism 182. In some examples, the drive mechanism 181 may drive the puncture mechanism 182. In some examples, the drive mechanism 181 may drive the puncture mechanism 182 to move towards the target 2. In some examples, the drive mechanism 181 may also drive the puncture mechanism 182 to move away from the target 2. In some examples, the drive mechanism 181 can drive the puncture mechanism 182 to penetrate the target 2 subcutaneously, and drive the puncture mechanism 182 away from the target 2 to withdraw the puncture mechanism 182 from the subcutaneous tissue of the target 2. In some examples, the drive mechanism 181 can be connected to the puncture mechanism 182.

In some examples, the puncture mechanism 182 can penetrate the target 2 subcutaneously carrying the first channel 121. In some examples, after the puncture mechanism 182 penetrates the target 2 subcutaneously, the first channel 121 can be placed subcutaneously in the target 2.

As described above, the number of drive sources 1811 can be one or more. For example, when there is one drive source 1811, one drive source 1811 can drive the puncture mechanism 182 to move towards the target 2 and drive the puncture mechanism 182 away from the target 2. For another example, when there are two drive sources 1811, one drive source 1811 can drive the puncture mechanism 182 to move towards the target 2, and the other drive source 1811 can drive the puncture mechanism 182 to move away from the target 2.

In some examples, the transmission member 1812 can provide driving force to the puncture mechanism 182.

In some examples, the drive source 1811 can be connected to the puncture mechanism 182 via the transmission member 1812.

In some examples, the application component 18 can include a guide mechanism 183 (see Figures 9A and 9B). In some examples, the guide mechanism 183 can be configured to provide a guide path. In some examples, the guide mechanism 183 can guide the first channel 121. In some examples, the first channel 121 can move along the guide path. In some examples, at least a portion of the first channel 121 can be movably connected to a guide rail.

In some examples, the guide mechanism 183 may extend towards the body surface of the target 2. In some examples, the guide mechanism 183 may be a guide rail.

In some examples, the application component 18 may include a locking mechanism. In some examples, the locking mechanism may be configured to lock the drive mechanism 181 before the target 2 uses the fluid delivery device 1. In some examples, locking the drive mechanism 181 may mean preventing the accumulated driving force of the drive mechanism 181 from being released. In some examples, the locking mechanism may release the drive mechanism 181, after which the drive mechanism 181 may release the accumulated driving force.

The following description uses an example in which the application component 18 includes both the drive mechanism 181 and the puncture mechanism 182.

In some examples, referring to Figure 9A, the transmission member 1812 may be two connecting blocks and two connecting rods. In some examples, one end of the two connecting rods can be movably connected to two connecting blocks, and the other end of the two connecting rods can be movably connected to the puncture mechanism 182.

In some examples, the drive source 1811 can be a tension spring. In some examples, two ends of the tension spring can be fixed to the two connecting blocks respectively.

In some examples, referring to Figure 9A, the tension spring can be in a compressed state when the locking mechanism locks the drive mechanism 181. In some examples, the puncture mechanism 182 can be manually inserted into the subcutaneous tissue of the target 2 along a first direction D1.

In some examples, referring to Figure 9B, after the puncture mechanism 182 is manually inserted into the subcutaneous tissue of the target 2, the locking mechanism can release the drive mechanism 181, and the tension spring can extend and provide a driving force, which can be an axial force. In some examples, the tension spring can push two connecting blocks to move in opposite directions along a third direction D3' and D3", respectively. The two connecting blocks pull the puncture mechanism 182 away from the target 2 along a second direction D2 by pulling two connecting rods. Thus, the first channel 121 can be placed subcutaneously in the target 2.

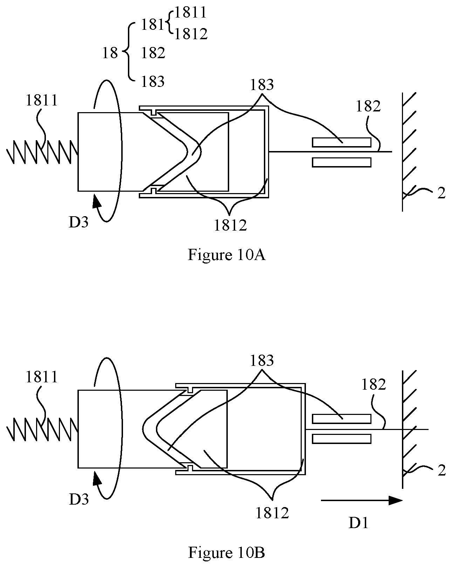

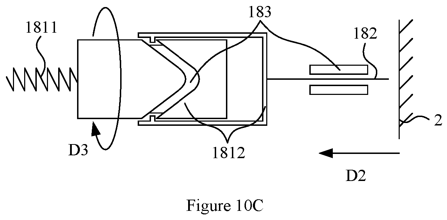

Figure 10A is a schematic diagram illustrating the puncture mechanism 182 before entering the target 2 in the second embodiment of the application component 18 according to an example of this disclosure. Figure 10B is a schematic diagram illustrating the puncture mechanism 182 entering the target 2 in the second embodiment of the application component 18 according to an example of this disclosure. Figure 10C is a schematic diagram illustrating the puncture mechanism 182 withdrawing from the target 2 in the second embodiment of the application component 18 according to an example of this disclosure.

In some examples, referring to Figure 10A, the transmission member 1812 can be a cylinder and a connecting rod. In some examples, two ends of the connecting rod can be movably connected to the cylinder, and the puncture mechanism 182 can be fixed to the connecting rod. In some examples, the connecting rod can be generally C-shaped.

In some examples, a portion of the guide mechanism 183 can be disposed in the cylinder. In some examples, the portion of the guide mechanism 183 disposed in the cylinder can be a groove. In some examples, two ends of the connecting rod can be located in the groove.

In some examples, the drive source 1811 can be a torsion spring. In some examples, one end of the torsion spring can be fixed to the cylinder. In some examples, the torsion spring can be fixed to the end of the cylinder away from the puncture mechanism 182.

In some examples, referring to Figure 10A, the torsion spring can be in a compressed state when the locking mechanism locks the drive mechanism 181.

In some examples, referring to Figure 10B, when the locking mechanism releases the drive mechanism 181, the torsion spring can extend and provide a driving force, which can be a circumferential force. In some examples, the cylinder can rotate in the third direction D3 under the action of the driving force, and the connecting rod can move towards the target 2 in the first direction D1 or away from the target 2 in the second direction D2 under the guidance of the groove.

In some examples, referring to Figure 10B, when the groove guides the connecting rod to move towards the target 2, the connecting rod pushes the puncture mechanism 182 to move towards the target 2 in the first direction D1 to pierce the target 2.

In some examples, referring to Figure 10C, after the puncture mechanism 182 pierces the target 2, the cylinder can continue to rotate along the third direction D3, and the grooved guide the connecting rod to move away from the target 2 along the second direction D2. The connecting rod pulls the puncture mechanism 182 away from the target 2 along the second direction D2. Thus, the first channel 121 can be placed subcutaneously in the target 2.

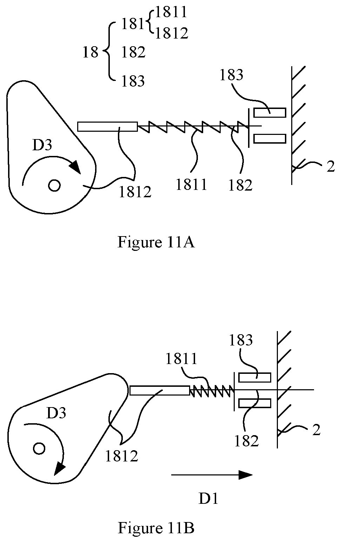

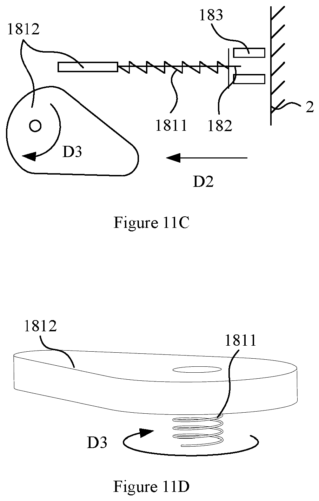

Figure 11A is a schematic diagram illustrating the puncture mechanism 182 before entering the target 2 in the third embodiment of the application component 18 according to an example of this disclosure. Figure 11B is a schematic diagram illustrating the puncture mechanism 182 entering the target 2 in the third embodiment of the application component 18 according to an example of this disclosure. Figure 11C is a schematic diagram illustrating the puncture mechanism 182 withdrawing from the target 2 in the third embodiment of the application component 18 according to an example of this disclosure. Figure 11D is a schematic diagram illustrating the drive source 1811 in the third embodiment of the application component 18 according to an example of this disclosure.

In some examples, referring to Figure 11A, the transmission member 1812 can be a cam and a push rod. In some examples, the drive source 1811 can be a torsion spring and a tension spring. In some examples, one end of the torsion spring can be fixed to the cam (see Figure 11D), and one end of the tension spring can be fixed to the push rod.

In some examples, when the locking mechanism locks the drive mechanism 181, the torsion spring can be in a compressed state, and the tension spring can be in a free state (see Figure 11A).

In some examples, referring to Figure 11B, when the locking mechanism releases the drive mechanism 181, the torsion spring can extend and provide the driving force, which can be a circumferential force. In some examples, the cam can rotate along the third direction D3 under the action of the driving force, thereby pushing the push rod and compressing the tension spring. In some examples, the cam can push the push rod, thereby pushing the puncture mechanism 182 to move towards the target 2 along the guiding direction (i.e., the first direction D1) of the guide mechanism 183 to pierce the target 2.

In some examples, referring to Figure 11C, after the puncture mechanism 182 pierces the target 2, the cam can continue to rotate along the third direction D3 and separate from the push rod. The tension spring is no longer compressed and extends to push the push rod towards the second direction D2, thereby pushing the puncture mechanism 182 away from the target 2 along the second direction D2. Thus, the first channel 121 can be placed subcutaneously in the target 2.

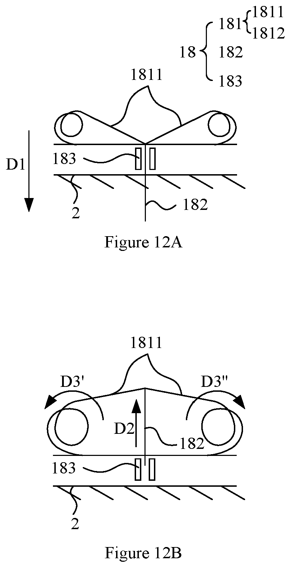

Figure 12A is a schematic diagram illustrating the puncture mechanism 182 entering the target 2 in a fourth embodiment of the application component 18 according to an example of this disclosure. Figure 12B is a schematic diagram illustrating the puncture mechanism 182 withdrawing from the target 2 in the fourth embodiment of the application component 18 according to an example of this disclosure.

In some examples, referring to Figure 12A or Figure 12B, the drive source 1811 can be a torsion spring. In some examples, one end of the torsion spring can be fixed to the puncture mechanism 182. The other end of the torsion spring can be fixed to the housing of the fluid delivery device 1.

In some examples, the number of torsion springs can be one or more.

In some examples, referring to Figure 12A, when the locking mechanism locks the drive mechanism 181, the torsion springs can be in a compressed or stretched state. In some examples, the puncture mechanism 182 can be manually inserted into the subcutaneous tissue of the target 2 along the first direction D1.

In some examples, referring to Figure 12B, after the puncture mechanism 182 is manually inserted into the subcutaneous tissue of the target 2, the locking mechanism can release the drive mechanism 181, and the torsion springs can extend and provide driving force. The directions of the driving forces of the two torsion springs can be the third direction D3' and D3", respectively. In some examples, guided by the guide mechanism 183, the torsion springs can pull the puncture mechanism 182 away from the target 2 along the second direction D2. Thus, the first channel 121 can be placed subcutaneously in the target 2.

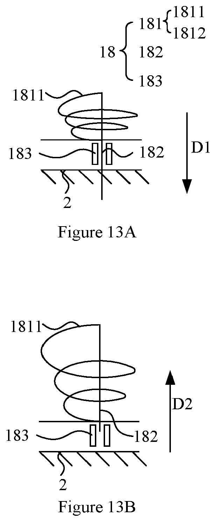

Figure 13A is a schematic diagram illustrating the puncture mechanism 182 entering the target 2 in a fifth embodiment of the application component 18 according to an example of this disclosure. Figure 13B is a schematic diagram illustrating the puncture mechanism 182 withdrawing from the target 2 in the fifth embodiment of the application component 18 according to an example of this disclosure.

In some examples, referring to Figure 13A or Figure 13B, the drive source 1811 can be a spiral spring. In some examples, one end of the spiral spring can be fixed to the puncture mechanism 182. The other end of the spiral spring can be fixed to the housing of the fluid delivery device 1.

In some examples, referring to Figure 13A, the spiral spring can be in a compressed or stretched state when the locking mechanism locks the drive mechanism 181. In some examples, the puncture mechanism 182 can be manually inserted into the subcutaneous tissue of the target 2 along the first direction D1.

In some examples, referring to Figure 13B, after the puncture mechanism 182 is manually inserted into the subcutaneous tissue of the target 2, the locking mechanism can release the drive mechanism 181, and the spiral spring can extend and provide a driving force, the direction of which can be the second direction D2. In some examples, guided by the guide mechanism 183, the spiral spring can pull the puncture mechanism 182 away from the target 2 along the second direction D2. Thus, the first channel 121 can be placed subcutaneously into the target 2.

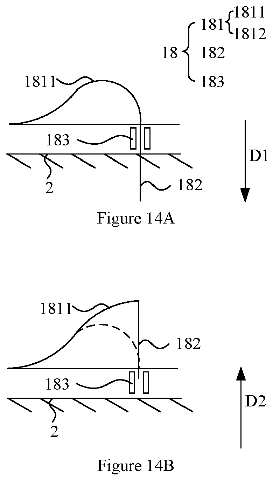

Figure 14A is a schematic diagram illustrating the puncture mechanism 182 entering the target 2 in a sixth embodiment of the application component 18 according to an example of this disclosure. Figure 14B is a schematic diagram illustrating the puncture mechanism 182 withdrawing from the target 2 in the sixth embodiment of the application component 18 according to an example of this disclosure.

In some examples, referring to Figure 14A or Figure 14B, the drive source 1811 can be a spring sheet. In some examples, one end of the spring sheet can be fixed to the puncture mechanism 182. The other end of the spring sheet can be fixed to the housing of the fluid delivery device 1.

In some examples, the number of spring sheets can be one or more.

In some examples, referring to Figure 14A, when the locking mechanism locks the drive mechanism 181, the spring sheet can be in a compressed or stretched state. In some examples, the puncture mechanism 182 can be manually inserted subcutaneously into the target 2 along the first direction D1.

In some examples, referring to Figure 14B, after the puncture mechanism 182 is manually inserted subcutaneously into the target 2, the locking mechanism can release the drive mechanism 181, and the spring sheet can extend and provide a driving force, the direction of which can be along the third direction D3. In some examples, guided by the guide mechanism 183, the spring sheet can pull the puncture mechanism 182 away from the target 2 along the second direction D2. Thus, the first channel 121 can be placed subcutaneously in the target 2.

According to this disclosure, the fluid delivery device 1 capable of quantitatively delivering fluid is provided.

Although the present disclosure has been specifically described above in conjunction with the accompanying drawings and examples, it is to be understood that the above description does not limit the present disclosure in any way. Those skilled in the art can make modifications and changes to the present disclosure as needed without departing from the essential spirit and scope of the present disclosure, and all such modifications and changes fall within the scope of the present disclosure.

## Claims

1. A fluid delivery device, **characterized in that** it comprises a fluid channel, an actuation component, and a flow limiting component, wherein the fluid channel includes a first channel, a reservoir, and a second channel, which are in fluid communication with each other; the actuation component is configured to provide actuating force to make fluid flow into or flow out of the reservoir; and the flow limiting component is configured to open or close the first channel and/or the second channel.

2. The fluid delivery device according to claim 1, **characterized in that** the first channel, the reservoir, and the second channel are sequentially connected.

3. The fluid delivery device according to claim 1, **characterized in that** the first channel, the reservoir, and the second channel are integrally formed.

4. The fluid delivery device according to claim 1, **characterized in that** the actuation component includes a power source configured to provide the actuating force.

5. The fluid delivery device according to claim 1, **characterized in that** the actuating force acts on the reservoir, and the reservoir actuates the fluid within it after being subjected to force.

6. The fluid delivery device according to claim 1, **characterized in that** the actuating force acts on the fluid.

7. The fluid delivery device according to claim 5, **characterized in that** the actuating force is configured to increase the pressure in the reservoir to make the fluid flow out of the reservoir.

8. The fluid delivery device according to claim 7, **characterized in that** the reservoir is deformed to reduce its volume.

9. The fluid delivery device according to claim 5, **characterized in that** the actuating force is configured to reduce the pressure in the reservoir to make the fluid flow into the reservoir.

10. The fluid delivery device according to claim 9, **characterized in that** the reservoir is deformed to increase its volume.

11. The fluid delivery device according to claim 1, **characterized in that** the actuating force acts on the reservoir to increase or decrease its volume.

12. The fluid delivery device according to claim 1, **characterized in that** the reservoir is made of an elastic material.

13. The fluid delivery device according to claim 1, **characterized in that** the flow limiting component restricts the flow of the fluid in the first channel and/or the second channel.

14. The fluid delivery device according to claim 1, **characterized in that** the flow limiting component comprises a valve disposed on a flow path of the fluid in the first channel and/or the second channel.

15. The fluid delivery device according to claim 1, **characterized in that** the flow limiting component acts on the first channel and/or the second channel to close or open the flow path of the fluid.

16. The fluid delivery device according to claim 1, **characterized in that** the actuation component provides the actuating force in response to the flow limiting component opening the first channel and/or the second channel.

17. The fluid delivery device according to claim 1, **characterized in that** the flow limiting component alternately opens the first channel and the second channel.

18. The fluid delivery device according to claim 1, **characterized in that** it further comprises a holding component configured to hold the position of the actuation component and provide the actuating force.
